# EUROPÄISCHE PATENTSCHRIFT

(11) **EP 2 906 191 B1**
(45) Veröffentlichungstag und Bekanntmachung des Hinweises auf die Patenterteilung: **28.03.2018**
(21) Anmeldenummer: 13770919.2
(22) Anmeldetag: 01.10.2013
(51) Int. Cl.: A61K 8/49, A61Q 5/10, C07D 239/50

(54) **HAARFÄRBEMITTEL ENTHALTEND AMINOPYRIMIDINDERIVATE**
HAIR DYE CONTAINING AMINOPYRIMIDINE DERIVATIVES
TEINTURE POUR LES CHEVEUX CONTENANT DES DÉRIVÉS DE L'AMINOPYRIMIDINE

(30) Priorität: 10.10.2012 DE 102012218459
(43) Veröffentlichungstag der Anmeldung: 19.08.2015
(73) Patentinhaber: Henkel AG & Co. KGaA, 40589 Düsseldorf (DE)
(72) Erfinder: GEBERT-SCHWARZWÄLDER, Antje, 41469 Neuss (DE); GIESA, Helmut, 40670 Meerbusch (DE); KOENEN, Annika, 41516 Grevenbroich (DE)
(86) Internationale Anmeldenummer: PCT/EP2013/070432
(87) Internationale Veröffentlichungsnummer: WO 2014/056760

(56) Entgegenhaltungen:
- WO-A1-2006/029712
- US-A- 4 046 503
- BOYLE P H ET AL: "SYNTHESIS AND PROPERTIES OF 7-ALKOXYFURAZANOÄ3,4-DÜPYRIMIDINES AND THEIR USE IN THE PREPARATION OF 4-ALKOXYPTERIDINE AND N3-SUBSTITUTED PTERINS", THE JOURNAL OF ORGANIC CHEMISTRY, AMERICAN CHEMICAL SOCIETY [NOT]ETC. , US, Bd. 50, Nr. 25, 1. Januar 1985 (1985-01-01), Seiten 5127-5132, XP008041325, ISSN: 0022-3263, DOI: 10.1021/JO00225A030

## Beschreibung

Die Erfindung betrifft ein Mittel zur Farbveränderung von keratinhaltigen Fasern, insbesondere menschlichen Haaren, welches bestimmte substituierte Aminopyrimidinderivate enthält.

Die Erfindung betrifft weiterhin bestimmte substituierte Aminopyrimidinderivate.

Die Veränderung von Form und Farbe der Haare stellt einen wichtigen Bereich der modernen Kosmetik dar. Zur modischen Farbgestaltung von Frisuren oder zur Kaschierung von ergrautem oder weißem Haar mit modischen oder natürlichen Farbtönen verwendet der Verbraucher farbverändernde Mittel.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung bzw. Farbveränderung diverse Systeme.

Für permanente, intensive Färbungen mit entsprechenden Echtheitseigenschaften werden
sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch intensive hervorragende, lang anhaltende Färbeergebnisse aus. Für natürlich wirkende Färbungen kann eine Mischung aus einer größeren Zahl von Oxidationsfarbstoffvorprodukten eingesetzt werden, wobei in vielen Fällen weiterhin zusätzlich direktziehende Farbstoffe zur Nuancierung verwendet werden.

Insbesondere oxidative Haarfärbemittel sind trotz ihrer vorteilhaften Färbeeigenschaften für den Anwender oftmals mit Nachteilen behaftet, so dass für Oxidationsfarbstoffvorprodukte ein stetiger Fortentwicklungsbedarf besteht.

Auf der Suche nach Oxidationsfarbstoffvorprodukten mit gutem Verträglichkeitsprofil wurden viele Verbindungen erforscht, die aber häufig unter anwendungstechnischen Problemen, insbesondere mangelndem Grauabdeckungsvermögen, leiden. Außerdem besteht trotz bereits hoch entwickelter Färbesysteme weiterhin Bedarf an Färbesystemen, die hervorragende Leuchtkraft und Intensität der Färbungen erreichen, gleichzeitig jedoch über eine sehr gute Haltbarkeit, sehr gute Echtheitseigenschaften und eine hervorragende Homogenität verfügen.

Insbesondere im Bereich der Rot- und Blaufärbungen besteht Verbesserungsbedarf.

Viele bekannte Färbesysteme, mit denen rote und blaue Farbnuancen erzielt werden können, weisen keine zufrieden stellenden Waschechtheiten und kein ausreichendes Egalisierungsvermögen auf.

Bei der Erzielung blauer Farbnuancen ist die Verbräunung der Blautöne nachteilig, die mitunter beobachtet werden konnte, wenn herkömmliches Triaminopyrimidin als Entwicklerkomponente benutzt wurde.

Aufgabe der vorliegenden Erfindung ist es daher, die oben genannten Nachteile oxidativer Haarfärbemittel herabzusenken. Die Färbemittel sollen intensive Färbungen mit hoher Farbigkeit und mit einer guten Beständigkeit gegenüber äußeren Einflüssen, insbesondere mit guter Lichtechtheit und Waschechtheit, erzeugen, welche auch nach mehrmaligem Shampoonieren der Haare keine Farbabschwächung oder Farbverschiebung erleiden. Darüber hinaus sollen die Färbungen möglichst wenig selektiv sein, d.h. auf unterschiedlich vorbehandeltem Haar möglichst gleichmäßige, einheitliche Färbeergebnisse erzielen. Daneben sollen die Färbemittel ein toxikologisch vorteilhaftes Profil besitzen. Eine weitere Aufgabe der Erfindung ist es, Oxidationsfarbstoffvorprodukte zu entwickeln, mit denen insbesondere rote und/oder blaue Farbnuancen erzielt werden können, die hervorragende Waschechtheiten und ein gutes Egalisierungsvermögen aufweisen. Insbesondere soll die Verbräunung von Blautönen vermieden werden.

Schließlich ist es wünschenswert, eine möglichst große Nuancierung der einzelnen Farbtöne zu erreichen.

Es wurde gefunden, dass sich bestimmte alkoxylierte Aminopyrimidinderivate als Oxidationsfarbstoffvorprodukte hervorragend zum Färben von keratinhaltigen Fasern eignen. Sie ergeben Ausfärbungen mit guten Echtheitseigenschaften, hoher Farbintensität und hervorragender Brillanz sowie exzellenter Grauabdeckung.

Triaminoalkoxypyrimidinderivate als Entwicklerkomponenten in oxidativen Haarfärbemitteln sind bekannt aus der Offenlegungsschrift DE 2516117.

Alkoxylierte Aminopyrimidinderivate entsprechend nachfolgender Formel (I) als Oxidationsfarbstoffvorprodukte sind bislang nicht bekannt.

Ein erster Gegenstand der Erfindung ist daher ein Mittel zum Färben keratinischer Fasern, insbesondere menschlicher Haare, welches dadurch gekennzeichnet ist, dass es in einem kosmetischen Träger mindestens eine Verbindung der Formel (I) und/oder ein physiologisch verträgliches Salz einer Verbindung der Formel (I) enthält, 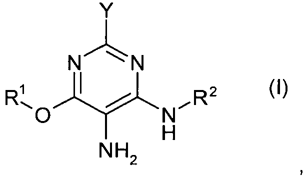 in der
- R¹ eine C₁-C₆-Hydroxyalkylgruppe, eine C₁-C₆-Alkoxygruppe, eine C₁-C₆-Alkoxy-C₁-C₆-hydroxyalkylgruppe, eine C₁-C₆-Alkoxy-C₂-C₆-alkylgruppe oder eine C₁-C₆-Polyalkoxygruppe sein kann,
- R² für ein Wasserstoffatom, und
- Y für eine NH₂-Gruppe steht.

Unter keratinischen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie beispielsweise Regeneratcellulose, Nitro-, Alkyl- oder Hydroxyalkyl- oder Acetylcellulose verwendet werden.

Die erfindungsgemäßen Mittel enthalten die Verbindungen der Formel (I) in einem kosmetischen Träger, bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind. Es ist aber auch denkbar, die Verbindungen gemäß Formel (I) in eine pulverförmige oder auch tablettenförmige Formulierung zu integrieren.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Bevorzugte Beispiele für eine C₁-C₆-Hydroxyalkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Erfiridungsgemäß bevorzugte C₁-C₆-Alkoxygruppen sind die Methoxy-, Ethoxy- oder die Propoxygruppe. Die 2-Methoxyethylgruppe, die 2-Ethoxyethylgruppe, die 3-Methoxypropylgruppe und die 3-Ethoxypropylgruppe sind Beispiele für eine C₁-C₆-Alkoxy-C₂-C₆-alkylgruppe, bevorzugt sind die 2-Methoxyethylgruppe und die 2-Ethoxyethylgruppe. Bevorzugte Beispiele für eine C₁-C₆-Alkoxy-C₂-C₆-hydroxyalkylgruppe sind die Gruppen Methoxymethanol, Methoxyethanol, Methoxypropan-1-ol, Methoxybutan-1-ol, 2-Ethoxymethanol, 2-Ethoxyethanol, 2-Ethoxypropan-1-ol oder 4-Ethoxybutan-1-ol. Erfindungsgemäß bevorzugte Beispiele für C₁-C₆-Polyalkoxygruppen sind die (Methoxymethoxy)methoxy-Gruppe, die 2-(2-Methoxyethoxy)ethoxy-Gruppe, die 3-(3-Methoxypropoxy)propoxy-Gruppe, die (Ethoxymethoxy)methoxy-Gruppe, die 2-(2-Ethoxyethoxy)ethoxy-Gruppe und die 3-(3-Ethoxypropoxy)propoxy-Gruppe.

Besonders bevorzugt steht der Rest R¹ für eine C₁-C₆-Hydroxyalkylgruppe, eine C₁-C₆- Polyalkoxygruppe oder eine C₁-C₆-Alkoxy-C₁-C₆-hydroxygruppe.

Bei den Verbindungen gemäß Formel (I) handelt es sich in einer besonders bevorzugten Ausführungsform um Derivate des 6-Hydroxy-triaminopyrimidins und damit um Aminoverbindungen. Aus diesen lassen sich in üblicher Weise die bekannten Säureadditionssalze herstellen. Alle Aussagen dieser Schrift und demgemäß der beanspruchte Schutzbereich beziehen sich daher sowohl auf die in freier Form vorliegenden Verbindungen als auch auf deren physiologisch verträgliche Salze organischer oder anorganischer Säuren. Beispiele für solche Salze sind die Hydrochloride, die Hydrobromide, die Sulfate, die Phosphate, die Acetate, die Propionate, die Citrate und die Lactate. Die Hydrochloride und die Sulfate sind dabei besonders bevorzugt. Erfindungsgemäß ganz besonders bevorzugt sind die Monohydrochloride, die Dihydrochloride und die Trihydrochloride.

Eine weitere bevorzugte Ausführungsform des ersten Erfindungsgegenstands ist dadurch gekennzeichnet, dass das Mittel mindestens eine Verbindung gemäß Formel (I) enthält, die ausgewählt ist aus

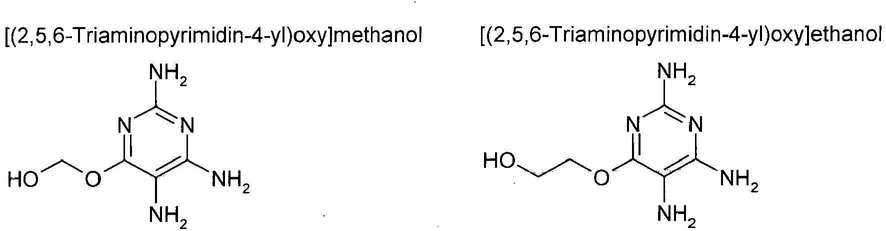

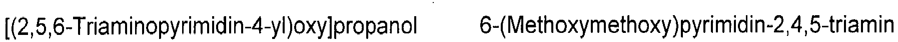

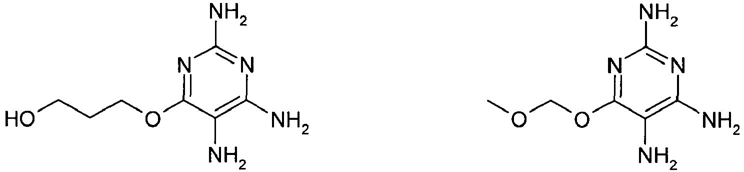

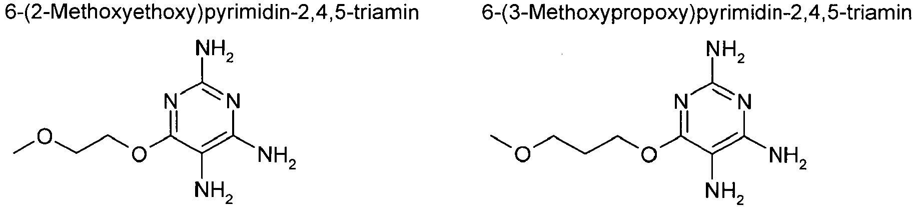

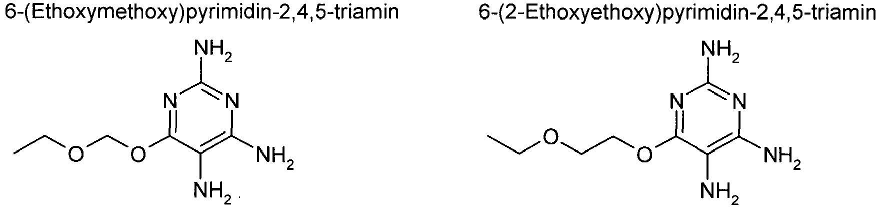

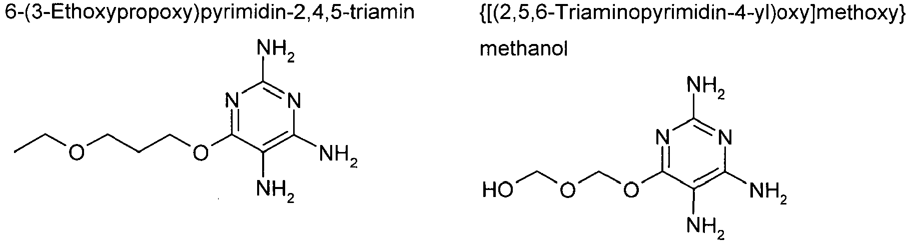

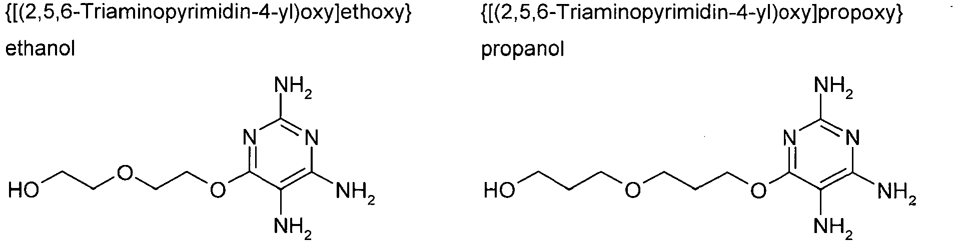

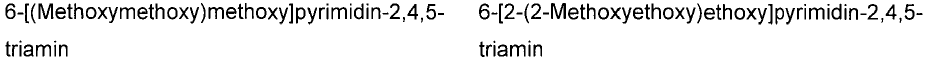

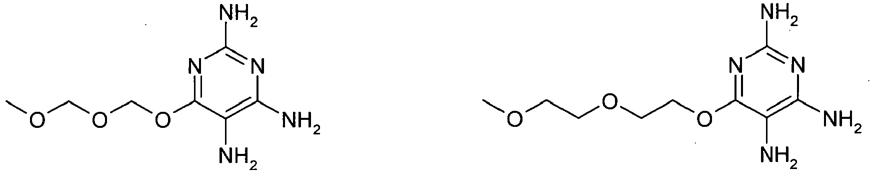

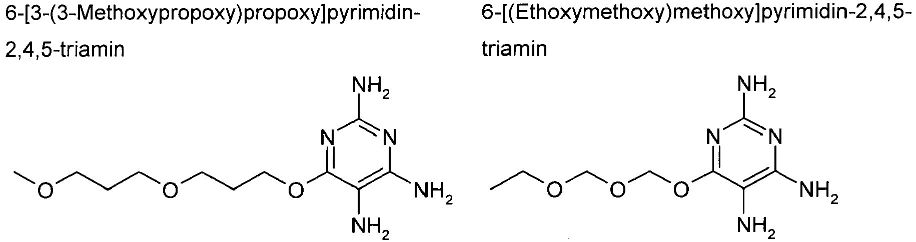

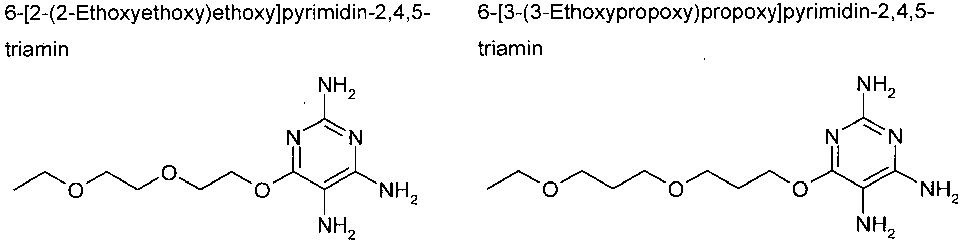

Erfindungsgemäß bevorzugte Mittel sind dadurch gekennzeichnet, dass sie die Verbindungen gemäß der Formel (I) und/oder deren physiologisch verträglichen Salze in einem Gewichtsanteil von 0,001 bis 5,0 Gew.-%, bevorzugt von 0,025 bis 2,5 Gew.-%, besonders bevorzugt von 0,05 bis 2,0 Gew.-% und insbesondere bevorzugt von 0,1 bis 1,5 Gew.-%, bezogen auf das Gesamtgewicht des anwendungsbereiten Mittels, enthalten.

Die Verbindungen der Formel (I) können als alleinige, farbverändernde Verbindungen im erfindungsgemäßen Mittel enthalten sein. Es ist erfindungsgemäß jedoch bevorzugt, wenn das Mittel zusätzlich mindestens ein Oxidationsfarbstoffvorprodukt vom Typ einer Kupplerkomponente enthält.

Kupplerkomponenten bilden im Rahmen der oxidativen Färbung allein keine signifikante Färbung aus, sondern benötigen stets die Gegenwart von Entwicklerkomponenten.

Kupplerkomponenten im Sinne der Erfindung erlauben mindestens eine Substitution eines chemischen Restes des Kupplers durch die oxidierte Form der Entwicklerkomponente. Dabei bildet sich kovalente Bindungen zwischen Kuppler- und Entwicklerkomponente aus.

Erfindungsgemäße Kupplerkomponenten werden bevorzugt als mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate, wie beispielsweise 6-Hydroxybenzomorpholin oder 6-Aminobenzomorpholin,
- Chinoxalinderivate, wie beispielsweise 6-Methyl-1,2,3,4-tetrahydrochinoxalin,

Gemische aus zwei oder mehreren Verbindungen aus einer oder mehreren dieser Klassen sind im Rahmen dieser Ausführungsform ebenso erfindungsgemäß.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-di-aminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, 5-Amino-4-chloro-o-kresol, 3-Amino-6-methoxy-2-methylaminopyridin, 5-Amino-4-chloro-o-kresol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Insbesondere bevorzugte Kupplerkomponenten sind 1,3-Bis-(2,4-diaminophenoxy)-propan, m-Aminophenol, Resorcin, 5-Amino-2-methylphenol, 2-Methylresorcin, 2-Chloro-6-methyl-3-aminophenol, 2,7-Dihydroxynaphthalin, 4-Chlorresorcin, 2,6-Dihydroxy-3,4-dimethylpyridin, 1-Amino-3-bis-(2-hydroxyethyl)aminobenzol (Lehmanns Blau), 2,4-Diaminophenoxyethanol, 5-Amino-4-chloro-o-kresol, 3-Amino-6-methoxy-2-methylaminopyridin, 5-Amino-4-chloro-o-kresol und/oder ein physiologisch verträgliches Salz dieser Verbindungen

Die Kupplerkomponenten werden in den erfindungsgemäßen Mitteln bevorzugt in einer Menge von 0,001 bis 5,0 Gew.-%, mehr bevorzugt von 0,025 bis 2,5 Gew.-%, besonders bevorzugt von 0,05 bis 2 Gew.-% und insbesondere von 0,1 bis 1,5 Gew.-%, jeweils bezogen auf das Gesamtgewicht des anwendungsbereiten Mittel, verwendet.

Die nachfolgenden Kombinationen eines erfindungsgemäßen Entwicklers mit ausgewählten Kupplern sind dabei besonders vorteilhaft:
2-[(2,5,6-Triaminopyrimidin-4-yl)oxy]ethanol und eine oder mehrere Verbindungen, ausgewählt aus 5-Amino-2-methylphenol, 1,3-Bis(2,4-diaminophenoxy)propan, Resorcin, 2-Methylresorcin, 2-Amino-3-hydroxypyridin, 3-Amino-2-methylamino-6-methoxypyridin, 2,7-Dihydroxynaphthalin.
6-[2-[2-Methoxyethoxy)ethoxy]pyrimidin-2,4,5-triamin und eine oder mehrere Verbindungen, ausgewählt aus 5-Amino-2-methylphenol, 1,3-Bis(2,4-diaminophenoxy)propan, Resorcin, 2-Methylresorcin, 2-Amino-3-hydroxypyridin, 3-Amino-2-methylamino-6-methoxypyridin, 2,7-Dihydroxynaphthalin.

In den voranstehend bevorzugten Kombinationen aus Oxidationsfarbstoffvorprodukten kann es erfindungsgemäß ebenfalls vorteilhaft sein, anstelle der ungeladenen Verbindung ein physiologisch verträgliches Salz dieser Verbindung einzusetzen.

Um eine ausgewogene und subtile Nuancenausbildung zu erzielen, ist es erfindungsgemäß vorteilhaft, wenn weitere farbgebende Komponenten im erfindungsgemäßen Mittel enthalten sind.

Es kann daher erfindungsgemäß bevorzugt sein, wenn das Mittel mindestens eine weitere farbgebende Komponente enthält, die ausgewählt ist aus zusätzlichen Oxidationsfarbstoffvorprodukten vom Entwicklertyp und/oder direktziehenden Farbstoffen.

Neben den Oxidationsfarbstoffvorprodukten vom Entwickler-Typ gemäß Formel (I) können die erfindungsgemäßen Mittel zusätzlich mindestens eine weitere Entwicklerkomponente enthalten.

Bevorzugte weitere Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diaza-cycloheptan, 1,10-Bis-(2,5-diaminophenyl)-1,4,7,10-tetraoxadecan, 4-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Besonders bevorzugte zusätzliche Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)-pyrazol sowie deren physiologisch verträglichen Salze.

Die zusätzlichen Entwicklerkomponenten werden bevorzugt in einer Menge von 0,0001 bis 10 Gew.-%, vorzugsweise 0,001 bis 5 Gew.-%, jeweils bezogen auf das anwendungsbereite Mittel, verwendet.

Weiterhin können die erfindungsgemäßen Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, insbesondere von 0,05 bis 5 Gew.-%, jeweils bezogen auf die gesamte Anwendungszubereitung, eingesetzt. Die Gesamtmenge an direktziehenden Farbstoffen beträgt vorzugsweise höchstens 3 Gew.-%.

Direktziehende Farbstoffe können in anionische, kationische und nichtionische direktziehende Farbstoffe unterteilt werden, die der Anforderungen der Trägerbasis vom Fachmann ausgewählt und eingesetzt werden.

Bevorzugte anionische direktziehende Farbstoffe sind die unter den internationalen Bezeichnungen bzw. Handelsnamen Bromphenolblau, Tetrabromphenolblau, Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehende Farbstoffe sind Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), Basic Blue 99, Basic Brown 16 und Basic Brown 17 sowie Yellow 87, Basic Orange 31 und Basic Red 51.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Die erfindungsgemäßen Mittel können neben der Verbindung gemäß Formel (I) auch naturanaloge Farbstoffe enthalten. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt. Besonders gut als Vorstufen naturanaloger Haarfarbstoffe geeignet sind Derivate des 5,6-Dihydroxyindolins, insbesondere 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure, sowie weiterhin Derivate des 5,6-Dihydroxyindols, insbesondere 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, sowie physiologisch verträgliche Salze der vorstehend genannten Verbindungen.

Im Falle der oxidativen Färbungen kann die Entwicklung der Farbe grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Als Oxidationsmittel kommen Persulfate, Peroxodisulfate, Chlorite, Hypochlorite und insbesondere Wasserstoffperoxid oder und/oder eines seiner festen Anlagerungsprodukte an organische oder anorganische Verbindungen in Frage.

Um eine vorzeitige, unerwünschte Reaktion der Oxidationsfarbstoffvorprodukte durch das Oxidationsmittel zu verhindern, werden Oxidationsfarbstoffvorprodukte und Oxidationsmittel selbst zweckmäßigerweise getrennt voneinander konfektioniert und erst unmittelbar vor der Anwendung in Kontakt gebracht.

In einer weiteren Ausführungsform der vorliegenden Erfindung sind daher Mittel bevorzugt, welche dadurch gekennzeichnet sind, dass sie unmittelbar vor der Anwendung durch Vermischen mindestens zweier Zubereitungen hergestellt wird, wobei die mindestens zwei Zubereitungen in mindestens zwei getrennt konfektionierten Containern bereitgestellt werden und wobei ein Container ein Färbemittel (A), welches in einem kosmetischen Träger mindestens ein Oxidationsfarbstoffvorprodukt gemäß Formel (I) enthält, und ein weiterer Container eine Oxidationsmittelzubereitung (B), enthaltend mindestens ein Oxidationsmittel, enthält.

Bevorzugt enthält die Oxidationsmittelzubereitung (B) als Oxidationsmittel Wasserstoffperoxid und/oder eines seiner festen Anlagerungsprodukte an organische oder anorganische Verbindungen, wie Harnstoff, Melamin sowie Natriumborat.

Bevorzugt beträgt die Menge an Oxidationsmittel im anwendungsbereiten Mittel 0,5 bis 12 Gew.-%, bevorzugt 2 bis 10 Gew.-% insbesondere bevorzugt zu 3 bis 6 Gew.-% (berechnet als 100 %-iges H₂O₂), jeweils bezogen auf das anwendungsbereite Mittel.

In einer weiteren bevorzugten Ausführungsform ist das erfindungsgemäße Mittel ein Mittel zum Färben und gegebenenfalls gleichzeitigen Aufhellen keratinischer Fasern, das bevorzugt 0,5 bis 15 Gew.-%, vorzugsweise 1 bis 12,5 Gew.-%, besonders bevorzugt 1,5 bis 10 Gew.-% und insbesondere 2 bis 6 Gew.-% Wasserstoffperoxid, jeweils bezogen auf das Gesamtgewicht des anwendungsbereiten Mittels, enthält.

Solche Oxidationsmittelzubereitungen sind vorzugsweise wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Erfindungsgemäß kann aber das Oxidationsfärbemittel auch zusammen mit einem Katalysator auf das Haar aufgebracht werden, der die Oxidation der Farbstoffvorprodukte aktiviert. Solche Katalysatoren sind z. B. bestimmte Enzyme, Iodide, Chinone oder Metallionen.

Weiterhin hat es sich als vorteilhaft erweisen, wenn die Oxidationsmittelzubereitungen mindestens einen Stabilisator oder Komplexbildner enthalten. Gebräuchliche und im Rahmen der vorliegenden Erfindung bevorzugte Komplexbildner und Stabilisatoren sind beispielsweise Polyoxycarbonsäuren, Polyamine, Ethylendiamintetraessigsäure (EDTA), N-Hydroxyethylethylendiamintriessigsäure, Diethylentriaminpentaessigsäure (DTPA), Ethylendiamindibernsteinsäure (EDDS), Hydroxyethyliminodiessigsäure, Nitridodiessigsäure-3-propionsäure, Isoserindiessigsäure, N,N-Di-(2-hydroxyethyl)glycin, N-(1,2-Dicarboxy-2-hydroxyethyl)glycin, N-(1,2-Dicarboxy-2-hydroxyethyl)asparaginsäure oder Nitrilotriessigsäure (NTA), Ethylendiamindiglutarsäure (EDGA), 2-Hydroxypropylendiamindibernsteinsäure (HPDS), Glycinamid-N,N'-dibernsteinsäure (GADS), Ethylendiamin-N-N'-diglutarsäure (EDDG), 2-Hydroxypropylendiamin-N-N'-dibernsteinsäure (HPDDS), Diaminoalkyldi-(sulfo-bernsteinsäure) (DDS), Ethylendicysteinsäure (EDC), Ethylendiamin-N-N'-bis(ortho-hydroxyphenyl)essigsäure (EDDHA), N-2-Hydroxyethylamin-N,N-diessigsäure, Glyceryliminodiessigsäure, Iminodiessigsäure-N-2-hydroxypropylsulfonsäure, Asparaginsäure-N-carboxymethyl-N-2,5-hydroxypropyl-3-sulfonsäure,β-Alanin-N,N'-diessigsäure, Asparaginsäure-N,N'-diessigsäure, Asparaginsäure-N-monoessigsäure, Dipicolinsäure, sowie deren Salze und/oder Derivate, geminale Diphosphonsäuren wie 1-Hydroxyethan-1,1-diphosphonsäure (HEDP), deren höhere Homologe mit bis zu 8 Kohlenstoffatomen sowie Hydroxy- oder Aminogruppen-haltige Derivate hiervon und 1-Aminoethan-1,1-diphosphonsäure, deren höhere Homologe mit bis zu 8 Kohlenstoffatomen sowie Hydroxy- oder Aminogruppen-haltige Derivate, Aminophosphonsäuren wie Ethylendiamintetra(methylenphosphonsäure) (EDTMP), Diethylen-triaminpenta(methylenphosphonsäure) (DTPMP) sowie deren höhere Homologe, oder Nitrilo-tri(methylenphosphonsäure), Phosphonopolycarbonsäuren wie 2-Phosphonobutan-1,2,4-tricarbonsäure, Cyclodextrine, sowie Alkalistannate (Natriumstannat), Alkalipyrophosphate (Tetranatriumpyrophosphat, Dinatriumpyrophosphat), Alkaliphosphate (Natriumphosphat), und Phosphorsäure sowie deren Salze.

Bei den erfindungsgemäß erforderlichen alkalischen pH-Werten der Behandlungslösungen liegen diese Komplexbildner zumindest teilweise als Anionen vor. Es ist unwesentlich, ob sie in Form der Säuren oder in Form von Salzen eingebracht werden. Im Falle des Einsatzes als Salze sind Alkali-, Ammonium- oder Alkylammoniumsalze, insbesondere Natriumsalze, bevorzugt.

Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Die Färbezubereitung und gegebenenfalls Oxidationsmittelzubereitung enthalten weitere Hilfs- und Zusatzstoffe. So hat es sich erfindungsgemäß als bevorzugt erwiesen, wenn die Färbezubereitung und/oder die Oxidationsmittelzubereitung mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Gemäß einer ersten bevorzugten Ausführungsform handelt es sich bei dem Verdickungsmittel um ein anionisches, synthetisches Polymer. Bevorzugte anionische Gruppen sind die Carboxylat- und die Sulfonatgruppe.

Beispiele für anionische Monomere, aus denen die polymeren anionischen Verdickungsmittel bestehen können, sind Acrylsäure, Methacrylsäure, Crotonsäure, Itaconsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind Maleinsäureanhydrid sowie insbesondere 2-Acrylamido-2-methylpropansulfonsäure und Acrylsäure.

Bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichnen Carbopol® im Handel erhältlich. Ebenfalls bevorzugt ist das Homopolymer der 2-Acrylamido-2-methylpropansulfonsäure, das beispielsweise unter der Bezeichnung Rheothik®11-80 im Handel erhältlich ist.

Innerhalb dieser ersten Ausführungsform kann es weiterhin bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Itaconsäuremono- und -diester, Vinylpyrrolidinon, Vinylether und Vinylester.

Die anionischen Acrylsäure- und/oder Methacrylsäure-Polymerisate oder -Copolymerisate sind in den erfindungsgemäßen Mitteln bevorzugt in einer Menge von 0,1 bis 10 Gew.-%, besonders bevorzugt von 1 bis 5 Gew.-%, jeweils bezogen auf das Gewicht des Mittels, enthalten.

Bevorzugte anionische Copolymere sind beispielsweise Copolymere aus Acrylsäure, Methacrylsäure oder deren C₁-C₆-Alkylestern, wie sie unter der INCI-Deklaration Acrylates Copolymere vertrieben werden. Ein bevorzugtes Handelsprodukt ist beispielsweise Aculyn® 33 der Firma Rohm & Haas. Weiterhin bevorzugt sind aber auch Copolymere aus Acrylsäure, Methacrylsäure oder deren C₁-C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20. Derartige Copolymere werden von der Firma Rohm & Haas unter der Handelsbezeichnung Aculyn® 22 sowie von der Firma National Starch unter den Handelsbezeichnungen Structure® 2001 und Structure® 3001 vertrieben.

Bevorzugte anionische Copolymere sind weiterhin Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie l'etraallyloxythan, Allylsucrose, Allylpentaerythrit und Methylen-bisacrylamid zum Einsatz kommen. Ein solches Polymer ist in den Handelsprodukten Sepigel®305 und Simulgel® 600 der Firma SEPPIC enthalten. Die Verwendung dieser Verbindungen, die neben der Polymerkomponente eine Kohlenwasserstoffmischung (C₁₃-C₁₄-Isoparaffin beziehungsweise Isohexadecan) und einen nichtionogenen Emulgator (Laureth-7 beziehungsweise Polysorbate-80) enthalten, hat sich im Rahmen der erfindungsgemäßen Lehre als besonders vorteilhaft erwiesen.

Auch Polymere aus Maleinsäureanhydrid und Methylvinylether, insbesondere solche mit Vernetzungen, sind bevorzugte Verdickungsmittel. Ein mit 1,9-Decadien vernetztes Maleinsäure-Methylvinylether-Copolymer ist unter der Bezeichnung Stabileze® QM im Handel erhältlich.

Bevorzugt kann das erfindungsgemäße Mittel zusätzlich mindestens ein anionisches Acrylsäure- und/oder Methacrylsäure-Polymerisat oder -Copolymerisat. Bevorzugte Polymerisate dieser Art sind:
- Polymerisate z.B. aus wenigstens 10 Gew.-% Acrylsäure-Niedrigalkylester, 25 bis 70 Gew.-% Methacrylsäure und ggf. bis zu 40 Gew.-% eines weiteren Comonomeren,
- Mischpolymerisate aus 50 bis 75 Gew.-% Ethylacrylat, 25 bis 35 Gew.-% Acrylsäure und 0 bis 25 Gew.-% anderer Comonomeren bekannt. Geeignete Dispersionen dieser Art sind im Handel erhältlich, z.B. unter der Handelsbezeichnung Latekoll® D (BASF).
- Copolymerisate aus 50 bis 60 Gew.-% Ethylacrylat, 30 bis 40 Gew.-% Methacrylsäure und 5 bis 15 Gew.-% Acrylsäure, vernetzt mit Ethylenglycoldimethacrylat.

Gemäß einer weiteren Ausführungsform handelt es sich bei dem Verdickungsmittel um einen kationisches synthetisches Polymer. Bevorzugte kationische Gruppen sind quartäre Ammoniumgruppen. Insbesondere solche Polymere, bei denen die quartäre Ammoniumgruppe über eine C₁-C₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (HP-1), 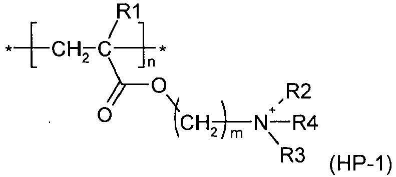 in der R1 = -H oder -CH₃ ist, R2, R3 und R4 unabhängig voneinander ausgewählt sind aus C₁-C₄-Alkyl-, -Alkenyl- oder -Hydroxyalkylgruppen, m = 1, 2, 3 oder 4, n eine natürliche Zahl und X- ein physiologisch verträgliches organisches oder anorganisches Anion ist, sowie Copolymere, bestehend im wesentlichen aus den in Formel (HP-1) aufgeführten Monomereinheiten sowie nichtionogenen Monomereinheiten, sind besonders bevorzugte kationische polymere Gelbildner. Im Rahmen dieser Polymeren sind diejenigen erfindungsgemäß bevorzugt, für die mindestens eine der folgenden Bedingungen gilt:
- R1 steht für eine Methylgruppe
- R2, R3 und R4 stehen für Methylgruppen
- m hat den Wert 2,

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloxy-ethyltrimethylammoniumChlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponente: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6) im Handel erhältlich.

Copolymere mit Monomereinheiten gemäß Formel (HP-1) enthalten als nichtionogene Monomereinheiten bevorzugt Acrylamid, Methacrylamid, Acrylsäure-C₁-C₄-Alkylester und Methacrylsäure-C₁-C₄-Alkylester. Unter diesen nichtionogenen Monomeren ist das Acrylamid besonders bevorzugt. Auch diese Copolymere können, wie im Falle der Homopolymeren oben beschrieben, vernetzt sein. Ein erfindungsgemäß bevorzugtes Copolymer ist das vernetzte Acrylamid-MethacroyloxyethyltrimethylammoniumChlorid-Copolymer. Solche Copolymere, bei denen die Monomeren in einem Gewichtsverhältnis von etwa 20:80 vorliegen, sind im Handel als ca. 50 %-ige nichtwässrige Polymerdispersion unter der Bezeichnung Salcare® SC 92 erhältlich.

In einer weiteren bevorzugten Ausführungsform werden natürlich vorkommende Verdickungsmittel eingesetzt. Bevorzugte Verdickungsmittel dieser Ausführungsform sind beispielsweise nichtionischen Guargums. Erfindungsgemäß können sowohl modifizierte als auch unmodifizierte Guargums zum Einsatz kommen. Nichtmodifizierte Guargums werden beispielsweise unter der Handelsbezeichnung Jaguar® C von der Firma Rhone Poulenc vertrieben. Erfindungsgemäß bevorzugte modifizierte Guargums enthalten C₁-C₆-Hydroxyalkylgruppen. Bevorzugt sind die Gruppen Hydroxymethyl, Hydroxyethyl, Hydroxypropyl und Hydroxybutyl. Derart modifizierte Guargums sind im Stand der Technik bekannt und können beispielsweise durch Reaktion der Guargums mit Alkylenoxiden hergestellt werden. Der Grad der Hydroxyalkylierung, der der Anzahl der verbrauchten Alkylenoxidmoleküle im Verhältnis zur Zahl der freien Hydroxygruppen der Guargums entspricht, liegt bevorzugt zwischen 0,4 und 1,2. Derart modifizierte Guargums sind unter den Handelsbezeichnungen Jaguar® HP8, Jaguar® HP60, Jaguar® HP120, Jaguarr® DC 293 und Jaguar® HP105 der Firma Rhone Poulenc im Handel erhältlich.

Weiterhin geeignete natürliche Verdickungsmittel sind ebenfalls bereits aus dem Stand der Technik bekannt.

Gemäß dieser Ausführungsform bevorzugt sind weiterhin Biosaccharidgums mikrobiellen Ursprungs, wie die Skleroglucangums oder Xanthangums, Gums aus pflanzlichen Exsudaten, wie beispielsweise Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen.

Bevorzugte Hydroxyalkylcellulosen sind insbesondere die Hydroxyethylcellulosen, die unter den Bezeichnungen Cellosize® der Firma Amerchol und Natrosol® der Firma Hercules vertrieben werden. Geeignete Carboxyalkylcellulosen sind insbesondere die Carboxymethylcellulosen, wie sie unter den Bezeichnungen Blanose® von der Firma Aqualon, Aquasorb® und Ambergum® von der Firma Hercules und Cellgon® von der Firma Montello vertrieben werden.

Bevorzugt sind weiterhin Stärke und deren Derivate. Stärke ist ein Speicherstoff von Pflanzen, der vor allem in Knollen und Wurzeln, in Getreide-Samen und in Früchten vorkommt und aus einer Vielzahl von Pflanzen in hoher Ausbeute gewonnen werden kann. Das Polysaccharid, das in kaltem Wasser unlöslich ist und in siedendem Wasser eine kolloidale Lösung bildet, kann beispielsweise aus Kartoffeln, Maniok, Bataten, Maranta, Mais, Getreide, Reis, Hülsenfrüchte wie beispielsweise Erbsen und Bohnen, Bananen oder dem Mark bestimmter Palmensorten (beispielsweise der Sagopalme) gewonnen werden. Erfindungsgemäß einsetzbar sind natürliche, aus Pflanzen gewonnene Stärken und/oder chemisch oder physikalisch modifizierte Stärken. Eine Modifizierung lässt sich beispielsweise durch Einführung unterschiedlicher funktioneller Gruppen an einer oder mehreren der Hydroxylgruppen der Stärke erreichen. Üblicherweise handelt es sich um Ester, Ether oder Amide der Stärke mit gegebenenfalls substituierten C₁-C₄₀-Resten. Besonders vorteilhaft ist eine mit einer 2-Hydroxypropylgruppe veretherte Maisstärke, wie sie beispielsweise von der Firma National Starch unter der Handelsbezeichnung Amaze® vertrieben wird.

Aber auch nichtionische, vollsynthetische Polymere, wie beispielsweise Polyvinylalkohol oder Polyvinylpyrrolidinon, sind als erfindungsgemäße Verdickungsmittel einsetzbar. Bevorzugte nichtionische, vollsynthetische Polymere werden beispielsweise von der Firma BASF unter dem Handelsnamen Luviskol® vertrieben. Derartige nichtionische Polymere ermöglichen, neben ihren hervorragenden verdickenden Eigenschaften, auch eine deutliche Verbesserung des sensorischen Gefühls der resultierenden Zubereitungen.

Als anorganische Verdickungsmittel haben sich Schichtsilikate (polymere, kristalline Natriumdisilicate) als besonders geeignet im Sinne der vorliegenden Erfindung erwiesen. Insbesondere Tone, insbesondere Magnesium Aluminium Silicate, wie beispielsweise Bentonit, besonders Smektite, wie Montmorillonit oder Hectorit, die gegebenenfalls auch geeignet modifiziert sein können, und synthetische Schichtsilikate, wie beispielsweise das von der Firma Süd Chemie unter der Handelsbezeichnung Optigel® vertriebene Magnesiumschichtsilikat, sind bevorzugt.

Zur weiteren Steigerung der Leistung der Oxidationsmittelzubereitung können der erfindungsgemäßen Zusammensetzung zusätzlich mindestens eine gegebenenfalls hydratisierte SiO₂-Verbindung zugesetzt. Es kann erfindungsgemäß bevorzugt sein, die gegebenenfalls hydratisierten SiO₂ Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt.

Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder besonders bevorzugt als Wasserglas eingesetzt. Diese SiO₂-Verbindungen können teilweise in wässriger Lösung vorliegen.

Erfindungsgemäß ganz besonders bevorzugt sind Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt. Bevorzugt sind Metasilikate, in denen das Verhältnis zwischen n und der Summe aus m und p bei 1:2 oder darunter liegt.

Neben den durch die Summenformel beschriebenen Komponenten können die Wassergläser in geringen Mengen noch weitere Zusatzstoffe, wie beispielsweise Phosphate oder Magnesiumsalze, enthalten.

Erfindungsgemäß besonders bevorzugte Wassergläser werden unter anderem von der Firma Henkel unter den Bezeichnungen Ferrosil® 119, Natronwasserglas 40/42, Portil® A, Portil® AW und Portil® W und von der Firma Akzo unter der Bezeichnung Britesil® C20 vertrieben.

Vorzugsweise sind die Zubereitung (A) und/oder gegebenenfalls die Oxidationsmittelzubereitung (B) als fließfähigen Zubereitungen konfektioniert.

Vorzugsweise wird den fließfähigen Zubereitungen (A) und/oder (B) weiterhin ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, amphoteren und nichtionischen Tensiden und Emulgatoren ausgewählt sind. Diese Stoffe werden nachfolgend ausführlich beschrieben.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel RO(CH₂CH₂O)ₓCH₂COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare α-Olefinsulfonate mit 8 bis 24 C-Atomen,
- Sulfonate ungesättigter Fettsäuren mit 8 bis 24 C-Atomen und 1 bis 6 Doppelbindungen,
- α-Sulfofettsäuremethylester von Fettsäuren mit 8 bis 30 C-Atomen,
- Alkylsulfate und Alkylethersulfate der Formel RO(CH₂CH₂O)ₓSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel 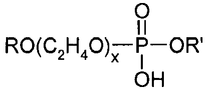
- in der R bevorzugt für einen aliphatischen, gegebenenfalls ungesättigten Kohlenwasserstoffrest mit 8 bis 30 Kohlenstoffatomen, R' für Wasserstoff, einen Rest (CH₂CH₂O)_{y}R und x und y unabhängig voneinander für eine Zahl von 1 bis 10 steht,
- sulfatierte Fettsäurealkylenglykolester der Formel RC(O)O(alkO)ₙSO₃H, in der R für einen linearen oder verzweigten, aliphatischen, gesättigten und/oder ungesättigten Alkylrest mit 6 bis 22 C-Atomen, alk für CH₂CH₂, CHCH₃CH₂ und/oder CH₂CHCH₃ und n für eine Zahl von 0,5 bis 5 steht,
- Monoglyceridsulfate und Monoglyceridethersulfate.

Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder SulfatGruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind das N-Kokosalkylaminopropionat, das Kokosacylaminoethylaminopropionat und das C₁₂-C₁₈-Acylsarcosin.

Weiterhin hat es sich als vorteilhaft erwiesen, wenn die erfindungsgemäßen Färbe- und Aufhellmittel weitere, nichtionogene grenzflächenaktive Stoffe, enthalten. Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 1 bis 50 Mol Ethylenoxid und/oder 0 bis 5 Mol Propylenoxid an lineare und verzweigte Fettalkohole mit 8 bis 30 C-Atomen, wie beispielsweise beispielsweise Lauryl-, Myristyl-, Cetyl-, aber auch Stearyl-, Isostearyl- und Oleylalkohol, an Fettsäuren mit 8 bis 30 C-Atomen und an Alkylphenole mit 8 bis 15 C-Atomen in der Alkylgruppe,
- mit einem Methyl- oder C₂-C₆-Alkylrest endgruppenverschlossene Anlagerungsprodukte von 1 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- Polyglycerinester und alkoxylierte Polyglycerinester, wie beispielsweise Poly(3)glycerindüsostearat (Handelsprodukt: Lameform®TGI (Henkel)) und Poly(2)glycerinpolyhydroxy-stearat (Handelsprodukt: Dehymuls®PGPH (Henkel)).
- Polyolfettsäureester, wie beispielsweise das Handelsprodukt Hydagen® HSP (Cognis) oder Sovermol-Typen (Cognis),
- höher alkoxylierte, bevorzugt propoxylierte und insbesondere ethoxylierte, Mono-, Di- und Triglyceride, wie beispielsweise Glycerinmonolaurat + 20 Ethylenoxid und Glycerinmonostearat + 20 Ethylenoxid,
- Aminoxide,
- Hydroxymischether,
- Sorbitanfettsäureester und Anlagerungeprodukte von Ethylenoxid an Sorbitanfettsäureester wie beispielsweise die Polysorbate und Sorbitanmonolaurat + 20 Mol Ethylenoxid (EO),
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylphenole und Alkylphenolalkoxylate mit 6 bis 21, insbesondere 6 bis 15 Kohlenstoffatomen in der Alkylkette und 1 bis 30 Ethylenoxid- und/oder Propylenoxid-Einheiten. Bevorzugte Vertreter dieser Klasse sind beispielsweise Nonylphenol + 9 EO und Octylphenol + 8 EO;
- Alkylpolyglykoside entsprechend der allgemeinen Formel RO-(Z)ₓ, wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.
   Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Die anionischen, nichtionischen, zwitterionischen oder amphoteren Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside stellen die quaternisierten Proteinhydrolysate dar.

Die Alkylamidoamine werden üblicherweise durch Amidierung natürlicher oder synthetischer Fettsäuren und Fettsäureschnitte mit Dialkylaminoaminen hergestellt und zeichnen sich neben einer guten konditionierenden Wirkung speziell durch ihre gute biologische Abbaubarkeit aus. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Ebenfalls sehr gut biologisch abbaubar sind quaternäre Esterverbindungen, sogenannte "Esterquats". Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform können nicht-ionische, zwitterionische und/oder amphotere Tenside sowie deren Mischungen bevorzugt sein.

In einer weiteren bevorzugten Ausführungsform kann die Wirkung des erfindungsgemäßen Wirkstoffes durch Emulgatoren gesteigert werden. Solche Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov® 68,
- Anlagerungsprodukte von 5 bis 60 Mol Ethylenoxid an Rizinusöl und gehärtetes Rizinusöl,
- Partialester von Polyolen mit 3 bis 6 Kohlenstoffatomen mit gesättigten Fettsäuren mit 8 bis 22 C-Atomen,
- Sterine, wobei als Sterine eine Gruppe von Steroiden verstanden wird, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide, vor allem Glucose-Phospolipide, die z. B. als Lecithine bzw. Phosphatidylcholine aus z.B. Eidotter oder Pflanzensamen (z.B. Sojabohnen) gewonnen werden,
- Fettsäureester von Zuckern und Zuckeralkoholen, wie Sorbit
- Polyglycerine und Polyglycerinderivate wie beispielsweise Polyglycerinpoly-12-hydroxystearat (Handelsprodukt Dehymuls® PGPH)
- Lineare und verzweigte Fettsäuren mit 8 bis 30 C-Atomen und deren Na-, K-, Ammonium-, Ca-, Mg- und Zn-Salze.

Die erfindungsgemäßen Mittel enthalten die Emulgatoren bevorzugt in Mengen von 0,1 bis 25 Gew.-%, insbesondere 0,5 bis 15 Gew.-%, bezogen auf die Gesamtmenge des anwendungsbereiten Mittel.

Nichtionogene Emulgatoren bzw. Tenside mit einem HLB-Wert von 10-15 können erfindungsgemäß besonders bevorzugt sein. Unter den genannten Emulgatoren-Typen können die Emulgatoren, welche kein Ethylenoxid und/oder Propylenoxid im Molekül enthalten ganz besonders bevorzugt sein.

Ferner können die erfindungsgemäßen Mittel weitere Wirk-, Hilfs- und Zusatzstoffe enthalten, wie beispielsweise
- nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane;
- Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare. Polysiloxan(A)-Polyoxyalkylen(B)-Blockcopolymere, gepfropften Silikonpolymere mit nicht silikonhaltigem, organischen Grundgerüst oder mit Polysiloxan-Grundgerüst, wie beispielsweise das unter der INCI-Bezeichnung Bis-PEG/PPG-20/20 Dimethicone vertriebene Handelsprodukt Abil B 8832 der Firma Degussa, oder deren Gemischen;
- kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol;
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere, Diallyldimethylammoniumchlorid/Acrylat-Copolymere, t-Butylaminoethylmethacrylat/N-(1,1,3,3-Tetramethylbutyl)acrylamid/Acrylat(/Methacrylat)-Copolymere,
- anionische Polymere wie beispielsweise Polyacrylsäuren, vernetzte Polyacrylsäuren, Vinylacetat/Crotonsäure-Copolymere, Vinylpyrrolidinon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-t-Butyl-acrylamid-Terpolymere,
- weitere Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline sowie Silikonöle,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Aminosäuren und Oligopeptide , insbesondere Arginin und/oder Serin,
- Proteinhydrolysate auf tierischer und/oder pflanzlicher Basis, wie beispielsweise Elastin-, Kollagen-, Keratin-, Seiden- und Milcheiweiß-Proteinhydrolysate, oder Mandel-, Reis-, Erbsen-, Kartoffel- und Weizenproteinhydrolysate, sowie in Form ihrer Fettsäure-Kondensationsprodukte oder gegebenenfalls anionisch oder kationisch modifizierten Derivate,
- pflanzliche Öle, beispielsweise Macadamianussöl, Kukuinussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl und Teebaumöl
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genusssäuren und Basen,
- Wirkstoffe wie Panthenol, Pantothensäure, Pantolacton, Allantoin, Pyrrolidinoncarbonsäuren und deren Salze sowie Bisabolol,
- Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole;
- Ceramide, bevorzugt die Sphingolipide wie Ceramide I, Ceramide II, Ceramide 1, Ceramide 2, Ceramide 3, Ceramide 5 und Ceramide 6, oder Pseudoceramide, wie insbesondere N-(C₈-C₂₂-Acyl)-(C₈-C₂₂-acyl)-hydroxyprolin,
- Vitamine, Provitamine und Vitaminvorstufen, insbesondere solche der Gruppen A, B₃, B₅, B₆, C, E, F und H,
- Pflanzenextrakte wie beispielsweise die Extrakte aus Aloe Vera, Angelica, Anis, Aprikose, Benzoe, Bergamotte, Birke, Brennnessel, Calmus, Cassis, Costus, Eibisch, Eichenrinde, Elemi, Estragon, Fichtennadel, Galbanum, Geranium, Ginseng, Grapefruit, Guajakholz, grünem Tee, Hamamelis, Hauhechel, Hopfen, Huflattich, Ingwerwurzel, Iris, Jasmin, Kamille, Kardamon, Klee, Klettenwurzel, Kiefer, Kiwi, Kokosnuss, Koriander, Kümmel, Latschen, Lavendel, Lemongras, Lilie, Limone, Lindenblüten, Litchi, Macis, Malve, Mandel, Mango, Melisse, Melone, Meristem, Myrrhe, Neroli, Olibanum, Opoponax, Orange, Patchouli, Petitgrain, Pinie, Quendel, Rooibos, Rosen, Rosmarin, Rosskastanie, Sandelholz, Salbei, Schachtelhalm, Schafgarbe, Sellerie, Tanne, Thymian, Wacholder, Weinblättern, Weißdorn, Weizen, Wiesenschaumkraut, Ylang-Ylang, Zeder und Zitrone.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O Dimethylether, CO₂ und Luft,
- Antioxidantien.

Die Auswahl dieser weiteren Stoffe wird der Fachmann gemäß der gewünschten Eigenschaften der Mittel treffen.

Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht der Anwendungsmischung, eingesetzt.

Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. Die erfindungsgemäßen Mittel können daher zusätzlich noch weitere Blondier- und/oder Bleichmittel enthalten.

Wird neben der Färbung der keratinischen Faser eine starke Aufhellung gewünscht, so ist daher es erfindungsgemäß bevorzugt, wenn zusätzlich eine Blondierzubereitung (C), enthaltend mindestens einen Bleichaktivator, der Mischung aus Oxidationsmittelzubereitung (B) und der Zubereitung (A), enthaltend mindestens ein Oxidationsfarbstoffvorprodukt gemäß Formel (I), beigemischt wird.

Es kann dabei unerheblich sein, ob zunächst eine Mischung aus (A) und (B) hergestellt wird, und anschließend die Blondierzubereitung (C) zugemischt wird, oder ob eine davon verschiedene Reihenfolge der Vermischung der einzelnen Komponenten genutzt wird. Es ist bevorzugt, die einzelnen Zubereitungen in möglichst naher zeitlicher Abfolge zu vermischen und das anwendungsbereite Mittel vorzugsweise zeitnah auf die keratinischen Fasern zu applizieren.

Eine weitere Ausführungsform der vorliegenden Anmeldung ist daher ein Mittel zum Bleichen und Färben keratinischer Fasern, dadurch gekennzeichnet, dass es durch Vermischen von mindestens einer Oxidationsmittelszubereitung (B), enthaltend mindestens ein Oxidationsmittel, ausgewählt aus Wasserstoffperoxid dessen Anlagerungsverbindungen an feste Träger, mindestens einer Blondierzubereitung (C), enthaltend mindestens einen Bleichkraftverstärker, und mindestens einer Zubereitung (A), enthaltend in einem kosmetischen Träger mindestens ein Oxidationsfarbstoffvorprodukt gemäß Formel (I), vor der Anwendung hergestellt wird.

In einer weiteren Ausführungsform ist es bevorzugt, wenn das erfindungsgemäße Färbemittel zusätzlich als Blondierzubereitung (C) mindestens eine anorganische Peroxoverbindung enthält. Vorzugsweise ist die anorganische Peroxoverbindung ausgewählt aus Ammoniumpersulfat, Alkalimetallpersulfaten, Ammoniumperoxomonosulfat, Alkalimetallhydrogenperoxomonosulfaten, Alkalimetallperoxodiphosphaten und Erdalkalimetallperoxiden. Besonders bevorzugte anorganische Peroxoverbindung als Bleichkraftverstärker sind Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid, insbesondere Ammoniumperoxodisulfat, Kaliumperoxodisulfat und Natriumperoxodisulfat.

Die anorganischen Peroxo-Verbindungen sind vorzugsweise in einer Menge von 0,1 bis 25 Gew.-%, insbesondere in einer Menge von 0,5 bis 15 Gew.-%, bezogen auf das Gesamtgewicht des anwendungsbereiten Mittels, enthalten.

Der Einsatz von Persulfatsalzen bzw. Peroxodisulfatsalzen erfolgt in der Regel in Form eines gegebenenfalls entstaubten Pulvers oder eines in Form gepressten Formkörpers.

Es kann jedoch erfindungsgemäß vorteilhaft sein, wenn die Mittel frei von anorganischen Peroxoverbindungen sind. Die erfindungsgemäßen Mittel können daher jedoch anstelle und/oder zusätzlich zu den festen Peroxoverbindungen einen weiteren Bleichkraftverstärker enthalten.

Als zusätzliche Bleichkraftverstärker können im Rahmen dieser Erfindung Verbindungen, die unter Perhydrolysebedingungen aliphatische Peroxocarbonsäuren, wie beispielsweise acylierte Alkylendiamine, insbesondere Tetraacetylethylendiamin (TAED), und/oder substituierte Perbenzoesäure ergeben, Kohlensäurederivate, insbesondere Ammoniumhydrogencarbonat, Ammoniumcarbonat, Natriumhydrogencarbonat, Natriumcarbonat, Kaliumhydrogencarbonat, Kaliumcarbonat und Calciumcarbonat, Alkylcarbonate und -carbamate sowie Silylcarbonate und -carbamate eingesetzt werden.

Die neben oder anstelle von Peroxoverbindungen eingesetzten Bleichkraftverstärker sind in den erfindungsgemäßen, kosmetischen Mitteln bevorzugt in Mengen von 0,05 bis 10 Gew.-%, insbesondere in Mengen von 0,2 bis 5 Gew.-%, jeweils bezogen auf das Gesamtgewicht des anwendungsbereiten Mittels, enthalten.

Obwohl prinzipiell keine Einschränkungen hinsichtlich der Formulierung der Blondierzubereitung (C) bestehen, hat es sich erfindungsgemäß als bevorzugt erwiesen, wenn die Zubereitung (C) wasserfrei formuliert ist.

Wasserfrei im Sinne der vorliegenden Erfindung bedeutet einen Wassergehalt bezogen auf die Zubereitung (C) von weniger als 5 Gew.-%, insbesondere von weniger als 2 Gew.-%. Blondierzubereitungen, die weniger als 0,1 Gew.-% Wasser, enthalten können erfindungsgemäß ganz besonders bevorzugt sein. Die Zubereitung (C) ist vorzugsweise als Pulver oder als wasserfreie Paste formuliert.

In einer weiteren, bevorzugten Ausführungsform kann das Mittel in der Zubereitung (C) als Bleichkraftverstärker mindestens ein kationisches Pyridinium-Derivat enthalten. Insbesondere sind erfindungsgemäße Mittel bevorzugt, die als kationisches Pyridinium-Derivat mindestens eine Verbindung aus 2-Acetyl-1-methylpyridinium-p-toluolsulfonat und/oder 4-Acetyl-1-methylpyridinium-p-toluolsulfonat und/oder N-Methyl-3,4-dihydroisochinolinium-p-toluolsulfonat.

Eine erfindungsgemäß bevorzugte Ausführungsform der vorliegenden Erfindung besteht darin, dass das anwendungsbereite Mittel einen pH- zwischen 7 und 11, insbesondere zwischen 8 und 10,5, insbesondere bevorzugt zwischen 8,5 und 10,0, besitzt.

Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren.

Bei den pH-Werten im Sinne der vorliegenden Erfindung handelt es sich um pH-Werte, die bei einer Temperatur von 22 °C gemessen wurden.

Erfindungsgemäß einsetzbare, organische Alkalisierungsmittel werden bevorzugt ausgewählt aus Alkanolaminen aus primären, sekundären oder tertiären Aminen mit einem C2-C₆-Alkylgrundkörper, der mindestens eine Hydroxylgruppe trägt. Besonders bevorzugte Alkanolamine werden ausgewählt aus der Gruppe 2-Aminoethan-1-ol (Monoethanolamin), 2-Amino-2-methylpropan-1-ol, 2-Amino-2-methyl-propan-1,3-diol und Triethanolamin.

Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein anorganisches Alkalisierungsmittel enthalten. Das erfindungsgemäße, anorganische Alkalisierungsmittel wird bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat. Ganz besonders bevorzugt sind Natriumhydroxid und/oder Kaliumhydroxid.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin, D/L-Arginin als ein Alkalisierungsmittel im Sinne der Erfindung eingesetzt.

Schließlich ist ein weiteres bevorzugtes Alkalisierungsmittel Ammoniak.

Bevorzugt werden die Alkalisierungsmittel in einer Menge von 0,05 bis 10 Gew.-%, insbesondere von 0,5 bis 5 Gew.-%, jeweils bezogen auf das Gesamtgewicht des anwendungsbereiten Mittels, enthalten.

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme bietet sich insbesondere dort an, wo Inkompatibilitäten der Inhaltsstoffe zu erwarten oder zu befürchten sind. Das anwendungsbereite Mittel wird bei solchen Systemen vom Verbraucher direkt vor der Anwendung durch Vermischen der Komponenten hergestellt. Ein Färbe- und/oder Aufhellungsmittel, bei dem die Oxidationsfarbstoffvorprodukte zunächst getrennt von der Oxidationsmittelzubereitung, enthaltend bevorzugt Wasserstoffperoxid, vorliegen, ist dabei bevorzugt.

Eine bevorzugte Darreichungsform des erfindungsgemäßen Mittels ist eine Verpackungseinheit (Kit-of-Parts), welche in getrennt voneinander konfektionierten Containern
- in einem Container A mindestens eine Zubereitung (A) enthält, enthaltend in einem kosmetischen Träger mindestens ein Oxidationsfarbstoffvorprodukt gemäß der Formel (I), und
- in einem Container B mindestens eine Oxidationsmittelzubereitung (B), enthaltend in einem kosmetischen Träger mindestens ein Oxidationsmittel,
enthält.

Wird ein besonders starker Aufhelleffekt gewünscht, so ist eine bevorzugte weitere Darreichungsform des erfindungsgemäßen Mittels eine Verpackungseinheit (Kit-of-Parts), welche in getrennt voneinander konfektionierten Containern
- in einem Container A mindestens eine Zubereitung (A), enthaltend in einem kosmetischen Träger mindestens ein Oxidationsfarbstoffvorprodukt gemäß der Formel (I),
- in einem Container B mindestens eine Oxidationsmittelzubereitung (B), enthaltend mindestens ein Oxidationsmittel, und
- in einem Container C mindestens eine Blondierzubereitung (C), enthaltend mindestens einen Bleichkraftverstärker, enthält.

Bevorzugt enthält die Mehrkomponentenverpackungseinheit (Kit-of-Parts) zusätzlich eine Gebrauchsanleitung. Darüber hinaus kann es bevorzugt sein, wenn weiterhin eine Applikationshilfe, wie beispielsweise ein Kamm oder ein Pinsel, und/oder eine persönliche Schutzausrüstung, wie beispielsweise Einweg-Handschuhe dem Kit beigefügt ist.

Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Das eigentliche Haarfärbemittel wird zweckmäßigerweise unmittelbar vor der Anwendung durch Mischung der Zubereitungen (A) mit (B) sowie gegebenenfalls (C) hergestellt. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Während der Einwirkzeit des Mittels auf der Faser kann es vorteilhaft sein, den Färbevorgang durch Wärmezufuhr zu unterstützen. Die Wärmezufuhr kann durch eine externe Wärmequelle, wie z.B. warme Luft eines Warmluftgebläses, als auch, insbesondere bei einer Haarfärbung am lebenden Probanden, durch die Körpertemperatur des Probanden erfolgen. Bei letzterer Möglichkeit wird üblicherweise die zu färbende Partie mit einer Haube abgedeckt. Insbesondere liegt die Temperatur während der Einwirkzeit zwischen 10 °C und 45 °C, insbesondere zwischen 20 °C und 40 °C. Die erfindungsgemäßen Färbemittel ergeben bereits bei physiologisch verträglichen Temperaturen von unter 45°C intensive Färbungen. Sie eignen sich deshalb besonders zum Färben von menschlichen Haaren.

Schließlich betrifft ein weiterer Gegenstand der vorliegenden Erfindung Verbindungen gemäß Formel (I) des ersten Erfindungsgegenstands. Bezüglich weiterer bevorzugter Ausführungsformen dieser Verbindungen gilt mutatis mutandis das zu den erfindungsgemäßen Mitteln Gesagte.

### Beispiele

### 1) Synthesebeispiele

### a) 2-[(2,5,6-Triaminopyrimidin-4-yl)oxy]ethanol, Dihydrochlorid

### (i) Synthese von 2-[(2,6-Diaminopyrimidin-4-yl)oxy]ethan-1-ol

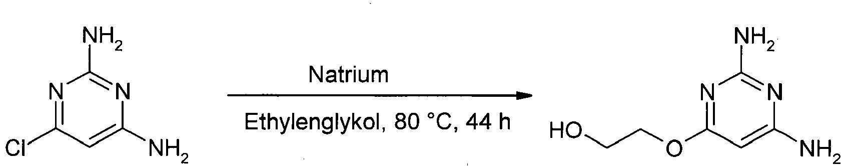

Zu einer Lösung von 4.6 g (0.20 mol) Natrium in 124 ml Ethylenglycol wurden 28.9 g (0.20 mol) 2,4-Diamino-6-chlorpyrimidin gegeben und unter Rühren für 44 h auf 80 °C erhitzt. Nach Beendigung der Reaktion wurde die Lösung mit 300 ml Wasser verdünnt und mit *tert*-Butylmethylether (5 x 200 ml) extrahiert. Die vereinigten org. Phasen wurden verworfen, aus der wässrigen Phasen fiel ein Niederschlag aus. Dieser wurde mit Wasser gewaschen, nach dem Trocknen erhielt man 2-[(2,6-Diaminopyrimidin-4-yl)oxy]ethan-1-ol (16.1 g, 47%) als hellbeigen Feststoff.
Smp.: 196-199 °C
¹H-NMR (300 MHz, d₆-DMSO): δ = 3,61 (t, 2 H, 2'-H₂), 4,09 (t, 2 H, 1'-H₂), 5,05 (s, 1 H, 5-H), 5,88 (s, 2 H, NH₂), 6,03 (s, 2 H, NH₂).
¹³C-NMR (125 MHz, d₆-DMSO): δ = 58,6 (2'-C), 65,6 (1'-C), 75,3 (5-C), 161,9 (2-C), 165,0 (4-C), 169,1 (6-C).

### (ii) Synthese von 2-[(2,6-Diamino-5-nitrosopyrimidin-4-yl)oxy]ethan-1-ol

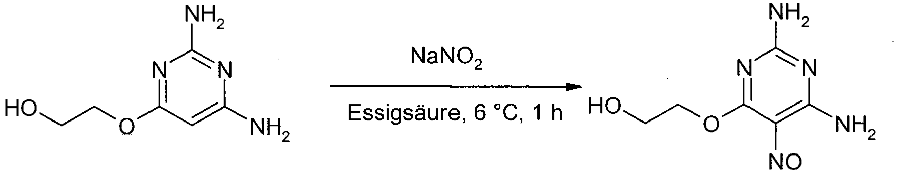

Zu einer Lösung von 8.50 g (0.050 mol) 2-[(2,6-Diaminopyrimidin-4-yl)oxy]ethan-1-ol in 100 ml Essigsäure (10%ig in Wasser) wurde bei 3-5 °C unter Rühren eine Lösung von 4.55 g (0.066 mol) Natriumnitrit in 20 ml Wasser über 1 h zugetropft. Anschließend wurde noch 2 h bei bei Raumtemperatur nachgerührt. Der entstandene Niederschlag wurde abgesaugt, mit Wasser nachgewaschen und getrocknet, wodurch man 2-[(2,6-Diamino-5-nitrosopyrimidin-4-yl)oxy]ethan-1-ol (6.71 g, 67%) als violetten Feststoff erhielt.
Smp.: 260 °C (Zers.)
¹H-NMR (300 MHz, d₆-DMSO): δ = 3,80 (t, 2H, 2'-H₂), 4,53 (t, 2H, 1'-H₂), 7,68 (s, 1H, NH₂), 7,75 (s, 1 H, NH₂), 8,04 (s, 1H. NH₂), 10,13 (s, 1H, NH₂).
¹³C-NMR (125 MHz, d₆-DMSO): δ = 59,3 (2'-C), 68,7 (1'-C), 139,6 (5-C), 151,0 (2-C), 163,6 (4-C), 171,0 (6-C).

### (iii) Synthese von 2-[(2,5,6-Triaminopyrimidin-4-yl)oxy]ethanol, Dihydrochlorid

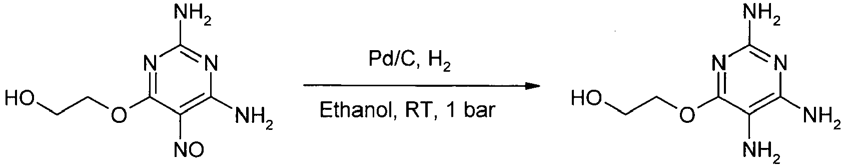

Zu einer Lösung von 6.57 g (0.033 mol) 2-[(2,6-Diamino-5-nitrosopyrimidin-4-yl)oxy]ethan-1-ol aus Stufe 4.2. in 400 ml Ethanol wurde 1.5 g (0,04 mol-%) Palladium auf Kohle (5%) gegeben und bei Raumtemperatur 19 h unter 1 Wasserstoffatmosphäre geschüttelt. Anschließend wurde das Reaktionsgemisch in 150 ml verdünnte Salzsäure (0.30 mol) gegossen, der Katalysator abfiltriert und das Filtrat fast bis zur Trockene eingeengt. Die ausgefallenen Kristalle wurden abgesaugt und mit *tert-*Butylmethylether nachgewaschen. Man erhielt 2-[(2,5,6-Triaminopyrimidin-4-yl)oxy]ethanol, Dihydrochlorid (7.17 g, 84%) als hellgelbe Kristalle.
Smp.: 176 °C (Zers.)
¹H-NMR (300 MHz, D₂O): δ = 3,91 (t, 2 H, 2'-H₂), 4,54 (t, 2 H, 1'-H₂).
¹³C-NMR (125 MHz, D₂O): δ = 62,5 (2'-C), 72,8 (1'-C), 87,2 (5-C), 153,9 (2-C), 157,0 (4-C), 168,3 (6-C).

### b) 6-[2-(2-Methoxyethoxy)ethoxy]pyrimidin-2.4.5-triamin, Dihydrochlorid

### (i) Synthese von 6-[2-(2-Methoxyethoxy)ethoxy]pyrimidin-2,4-diamin

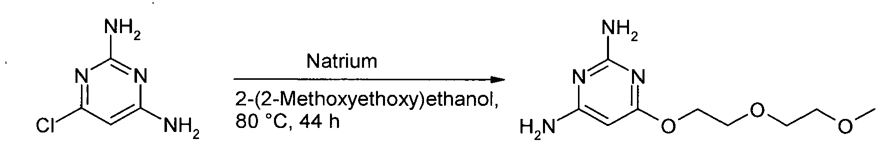

Zu einer Lösung von 4.6 g (0.20 mol) Natrium in 178 ml 2-(2-Methoxyethoxy)ethanol wurden 28.9 g (0.20 mol) 2,4-Diamino-6-chlorpyrimidin gegeben und unter Rühren für 48 h auf 80 °C erhitzt Nach Beendigung der Reaktion wurde das ausgefallene NaCl abgetrennt und die Lösung zur Trockene eingeengt. Der Rückstand wurde mehrfach in Ethanol aufgenommen und wieder eingeengt, um restliches 2-(2-Methoxyethoxy)ethanol zu entfernen. Nach dem Trocknen erhielt man 6-[2-(2-Methoxyethoxy)ethoxy]pyrimidin-2,4-diamin (49.8 g, 109%) als braunes Öl, das als Verunreinigung noch 2-(2-Methoxyethoxy)ethanol enthielt.
¹H-NMR (300 MHz, d₆-DMSO): δ = 3,20 (s, 3 H, 5'-H₃), 3,26 - 3,58 (m, 4 H, 3'-H₂, 4'-H₂), 3,62 (t, 2 H, 2'-H₂), 5,07 (s, 1 H, 5-H), 5,90 (s, 2 H, NH₂), 6,01 (s, 2 H, NH₂).
¹³C-NMR (125 MHz, d₆-DMSO): δ = 56,4 (5'-C), 69,2 (4'-C), 70,0 (3'-C), 71,6 (2'-C), 72,6 (1'-C), 76,6 (5-C), 163,2 (2-C), 166,3 (4-C), 170,2 (6-C).

### (ii) Synthese von 6-[2-(2-Methoxyethoxy)ethoxy]-5-nitrosopyrimidin-2,4-diamin

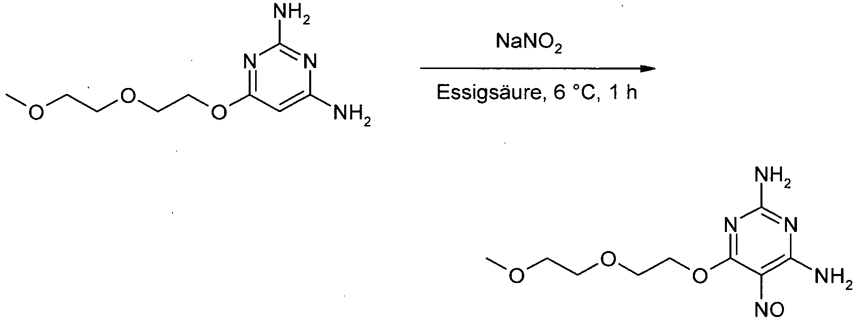

Zu einer Lösung von 22.8 g (0.100 mol) 6-[2-(2-Methoxyethoxy)ethoxy]pyrimidin-2,4-diamin in 200 ml Essigsäure (10%ig in Wasser) wurde bei 3-5 °C unter Rühren eine Lösung von 9.11 g (0.013 mol) Natriumnitrit in 40 ml Wasser über 1 h zugetropft. Anschließend wurde noch 2 h bei bei Raumtemperatur nachgerührt. Der entstandene Niederschlag wurde abgesaugt, mit Wasser nachgewaschen und getrocknet, wodurch man 6-[2-(2-Methoxyethoxy)ethoxy]-5-nitrosopyrimidin-2,4-diamin (11.7 g, 45%) als violetten Feststoff erhielt.
Smp.: 163-168 °C (Zers.)
¹H-NMR (300 MHz, d₆-DMSO): δ = 3,26 (s, 3H, 5'-H₃), 3,50 (t, 2H, 4'-H₂), 3,64 (t, 2H, 3'-H₂), 3,86 (t, 2H, 2'-H₂), 4,66 (t, 2H, 1'-H₂), 7,80 (s, 1H, NH₂), 7,85 (s, 1 H, NH₂), 8,09 (s, 1H, NH₂), 10,11 (s, 1H, NH₂). ¹³C-NMR (125 MHz, d₆-DMSO): δ = 57,1 (5'-C), 65,1 (4'-C), 67,5 (3'-C), 68,8 (2'-C), 70,3 (1'-C), 138,5 (5-C), 149,9 (2-C), 162,4 (4-C), 169,8 (6-C).

### (iii) Synthese von 6-[2-(2-Methoxyethoxy)ethoxy]pyrimidin-2,4,5-triamin, Dihydrochlorid

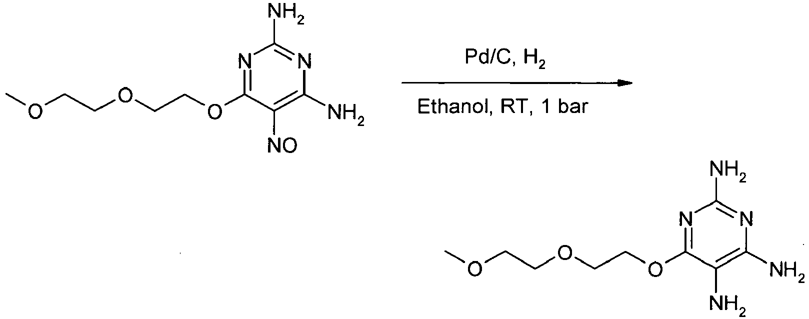

Zu einer Lösung von 11.3 g (0.044 mol) 6-[2-(2-Methoxyethoxy)ethoxy]-5-nitrosopyrimidin-2,4-diamin aus Stufe 5.2. in 400 ml Ethanol wurde 1.5 g (0,04 mol-%) Palladium auf Kohle (5%) gegeben und bei Raumtemperatur 16 h unter 1Wasserstoffatmosphäre geschüttelt. Anschließend wurde das Reaktionsgemisch in 80 ml verdünnte Salzsäure (0.23 mol) gegossen, der Katalysator abfiltriert und das Filtrat fast bis zur Trockene eingeengt. Die ausgefallenen Kristalle wurden abgesaugt und mit Ethanol nachgewaschen. Man erhielt 6-[2-(2-Methoxyethoxy)ethoxy]pyrimidin-2,4,5-triamin, Dihydrochlorid (11.7 g, 84%) als weiße Kristalle.
Smp.: 177-180 °C
¹H-NMR (300 MHz, CDCl₃): δ = 0,73 (s, 3 H, 5'-H₃), 1,00 (t, 2 H, 4'-H₂), 1,10 (t, 2 H, 3'-H₂), 1,26 (t, 2 H, 2'-H₂), 1,97 (t, 2 H, 1'-H₂).
¹³C-NMR (125 MHz, CDCl₃): δ = 66,0 (5'-C), 66,1 (4'-C), 66,8 (3'-C), 67,7 (2'-C), 69,3 (1'-C), 82,6 (5-C), 149,9 (2-C), 152,1 (4-C), 163,6 (6-C).

### 2) Ausfärbungen

### Herstellung der Färbecreme

Es wurden eine Färbecreme der folgenden Zusammensetzung hergestellt:

| | |
|---|---|
| Hydrenol® D¹ | 8,5 Gew.-% |
| Lorol® tech.² | 2,0 Gew.-% |
| Texapon® NSO³ | 20,0 Gew.-% |
| Dehyton® K⁴ | 12,5 Gew.-% |
| Eumulgin® B2⁵ | 0,75 Gew.-% |
| Natriumsulfit | 1,0 Gew.-% |
| Ammoniumsulfat | 1,0 Gew.-% |
| Entwicklerkomponente | 3 mmol |
| Kupplerkomponente | 3 mmol |
| Wasser | ad 100 |

| | |
|---|---|
| ¹ C₁₆₋₁₈-Fettalkohol (INCI-Bezeichnung: Cetearyl alcohol) (Cognis) ² C₁₂₋₁₈-Fettalkohol (INCI-Bezeichnung: Coconut alcohol) (Cognis) ³ Laurylethersulfat, Natriumsalz (ca. 27,5% Aktivsubstanz; INCI-Bezeichnung: Sodium Laureth Sulfate) (Cognis) ⁴ N,N-Dimethyl-N-(C₈₋₁₈-kokosamidopropyl)ammoniumacetobetain (ca. 30% Aktivsubstanz; INCI-Bezeichnung: Aqua (Water), Cocamidopropyl Betaine) (Cognis) ⁵ Cetylstearylalkohol mit ca. 20 EO-Einheiten (INCI-Bezeichnung: Ceteareth-20) (Cognis) | |

Hydrenol D und Lorol techn. wurden zusammen mit Texapon NSO, Dehyton K und Eumulgin B2 bei 80 °C aufgeschmolzen. Dann wurde die Schmelze mit dem in einem Teil des Wassers gelösten Natriumsulfit und Ammoniumsulfat emulgiert. Der erfindungsgemäße Entwickler wurde in einem weiteren Teil der angegebenen Wassermenge unter Erhitzen gelöst und unter Rühren hinzu gegeben. Der Kuppler wurde ebenfalls in einem Teil der angegebenen Wassermenge gelöst und unter Rühren hinzu gegeben. Dann wurde die Formulierung auf 100 % mit Wasser aufgefüllt und kalt gerührt.

Die auf diese Weise erhaltene Färbecreme wurde im Verhältnis 1:1 mit der folgenden Entwicklerdispersion mit einem Wasserstoffperoxidgehalt von 6% vermischt.

| | |
|---|---|
| Dipicolinsäure | 0,1 Gew.-% |
| Natriumpyrophosphat | 0,03 Gew.-% |
| Turpinal® SL⁶ | 1,50 Gew.-% |
| Texapon® N28⁷ | 2,00 Gew.-% |
| Acrysol® 22⁸ | 0,60 Gew.-% |
| Wasserstoffperoxid, 50 %ig | 6,00 Gew.-% |
| Natronlauge, 45%ig | 0,80 Gew.-% |
| Wasser | ad 100 Gew.-% |

| | |
|---|---|
| ⁶ 1-Hydroxyethan-1,1-diphosphonsäure (ca. 58 - 61% Aktivsubstanzgehalt; INCI-Bezeichnung: Etidronic Acid, Aqua (Water)) (Solutia) ⁷ Laurylethersulfat-Natrium-Salz (mind. 26,5 % Aktivsubstanzgehalt; INCI-Bezeichnung: Sodium Laureth Sulfate) (Cognis) ⁸ Acrylpolymer (ca. 29.5 - 30.5% Festkörper in Wasser; INCI-Bezeichnung: Acrylates/Steareth-20 Methacrylate Copolyme) | |

Für den Färbeprozess wurde jeweils auf eine Strähne zu 80 % ergrauten Haares (Kerling) die 4-fache Menge der anwendungsbereiten Mischung appliziert. Nach einer Einwirkzeit von 30 Minuten bei 32 °C wurden die Strähnen ausgespült und mit einem üblichen Haarwaschmittel ausgewaschen. Die Färbung der Strähnen wurde nach dem Trocknen visuell unter der Tageslichtlampe beurteilt. Die Färbeergebnisse sind in der nachfolgenden Tabelle zusammengefasst.
a) Ausfärbungen mit **2-[(2,5,6-Triaminopyrimidin-4-yl)oxy]ethanol,** Dihydrochlorid

| **Beispiel** | **Kupplerkomponente** | **Erhaltene Nuance/ Farbintensität** |
|---|---|---|
| **1** | Resorcin | pompejanischrot / +++ |
| **2** | 3-Amino-2-methylamino-6-methoxypyridin | biberbraun / +++ |
| **3** | 5-Amino-2-methylphenol | violettbraun / ++ |
| **4** | 3-Amino-2-hydroxypyridin | lehmfarben / + |
| **5** | 1,3-Bis(2,4-diaminophenoxy)propan | dunkelblau / +++ |
| **6** | 2,7-Dihydroxynaphthalin | karamellbraun / + |
| **7** | 2-Methylresorcin | kirschrot / +++ |
| +++ hohe Intensität | ++ mittlere Intensität | + niedrige Intensität |

**b) Ausfärbungen mit 6-[2-(2-Methoxyethoxy)ethoxy]pyrimidin-2,4,5-triamin, Dihydrochlorid**

| **Beispiel** | **Kupplerkomponente** | **Erhaltene Nuance/ Farbintensität** |
|---|---|---|
| **1** | Resorcin | bronzerot / +++ |
| **2** | 3-Amino-2-methylamino-6-methoxypyridin | olivbraun / +++ |
| **3** | 5-Amino-2-methylphenol | graurot / ++ |
| **4** | 3-Amino-2-hydroxypyridin | goldblond / + |
| **5** | 1,3-Bis(2,4-diaminophenoxy)propan | blaugrau / +++ |
| **6** | 2,7-Dihydroxynaphthalin | braunorange / + |
| **7** | 2-Methylresorcin | braunrot / +++ |
| +++ hohe Intensität | ++ mittlere Intensität | + niedrige Intensität |

## Patentansprüche

1. Mittel zum Färben von keratinischen Fasern, insbesondere menschlichen Haaren, **dadurch gekennzeichnet, dass** es in einem kosmetischen Träger mindestens eine Verbindung der Formel (I) und/oder ein physiologisch verträgliches Salz einer Verbindung der Formel (I) enthält, 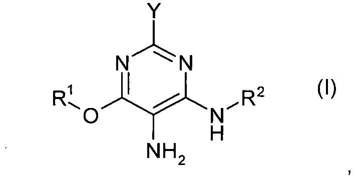 in der
R¹ für eine C₁-C₆-Hydroxyalkylgruppe, eine C₁-C₆-Alkoxygruppe, eine C₁-C₆-Alkoxy-C₁-C₆-hydroxyalkylgruppe, eine C₁-C₆-Alkoxy-C₂-C₆-alkylgruppe, eine C₁-C₆-Polyalkoxygruppe sein kann,
R² für ein Wasserstoffatom und
Y für eine NH2-Gruppe steht.

2. Mittel nach einem der Ansprüche 1 oder 2, **dadurch gekennzeichnet, dass** es mindestens eine Verbindung der Formel (I) enthält, die ausgewählt ist aus der Gruppe, die gebildet wird aus [(2,5,6-Triaminopyrimidin-4-yl)oxy]methanol, [(2,5,6-Triaminopyrimidin-4-yl)oxy]ethanol [(2,5,6-Triaminopyrimidin-4-yl)oxy]propanol, 6-(Methoxymethoxy)pyrimidin-2,4,5-triamin, 6-(2-Methoxyethoxy)pyrimidin-2,4,5-triamin, 6-(3-Methoxypropoxy)pyrimidin-2,4,5-triamin, 6-(Ethoxymethoxy)pyrimidin-2,4,5-triamin, 6-(2-Ethoxyethoxy)pyrimidin-2,4,5-triamin, 6-(3-Ethoxypropoxy)pyrimidin-2,4,5-triamin, {[(2,5,6-Triaminopyrimidin-4-yl)oxy]methoxy}methanol, {[(2,5,6-Triaminopyrimidin-4-yl)oxy]ethoxy}ethanol, {[(2,5,6-Triaminopyrimidin-4-yl)oxy]propoxy}propanol, 6-[(Methoxymethoxy)methoxy]pyrimidin-2,4,5-triamin, 6-[2-(2-Methoxyethoxy)ethoxy]pyrimidin-2,4,5-triamin, 6-[3-(3-Methoxypropoxy)propoxy]pyrimidin-2,4,5-triamin, 6-[(Ethoxymethoxy)methoxy]pyrimidin-2,4,5-triamin, 6-[2-(2-Ethoxyethoxy)ethoxy]pyrimidin-2,4,5-triamin, 6-[3-(3-Ethoxypropoxy)propoxy]pyrimidin-2,4,5-triamin sowie deren physiologisch verträglichen Salzen.

3. Mittel nach Anspruch 2, **dadurch gekennzeichnet, dass** es 2-[(2,5,6-Triaminopyrimidin-4-yl)oxy]ethanol und/oder ein physiologisch verträgliches Salz davon enthält.

4. Mittel nach Anspruch 2, **dadurch gekennzeichnet, dass** es 6-[2-(2-Methoxyethoxy) ethoxy]pyrimidin-2-4,5-triamin und/oder ein physiologisch verträgliches Salz davon enthält.

5. Mittel nach einem der Ansprüche 1 bis 4, **dadurch gekennzeichnet, dass** es die Verbindung(en) der Formel (I) und/oder deren physiologisch verträglichen Salze in einem Gewichtsanteil von 0,001 bis 5,0 Gew.-%, bevorzugt von 0,025 bis 2,5 Gew.-%, besonders bevorzugt von 0,05 bis 2,0 Gew.-% und insbesondere bevorzugt von 0,1 bis 1,5 Gew.-%, jeweils bezogen auf des Gesamtgewicht des anwendungsbereiten Mittels, enthält.

6. Mittel nach einem der Ansprüche 1 bis 5, **dadurch gekennzeichnet, dass** es 0,001 bis 5,0 Gew.-%, bevorzugt von 0,025 bis 2,5 Gew.-%, besonders bevorzugt von 0,05 bis 2,0 Gew.-% und insbesondere bevorzugt von 0,1 bis 1,5 Gew.-% mindestens einer Kupplerkomponente enthält, wobei sich die Mengenangaben auf das Gesamtgewicht des anwendungsbereiten Mittels beziehen.

7. Mittel nach Anspruch 6, **dadurch gekennzeichnet, dass** es als Kupplerkomponente mindestens eine der folgenden Verbindungen enthält: 1,3-Bis-(2,4-diaminophenoxy)-propan, m-Aminophenol, Resorcin, 5-Amino-2-methylphenol, 2-Methylresorcin, 2-Chloro-6-methyl-3-aminophenol, 2,7-Dihydroxynaphthalin, 4-Chlorresorcin, 2,6-Dihydroxy-3,4-dimethylpyridin, 1-Methoxy-2amino-4-β-hydroxy-ethylamino-benzen (Lehmanns Blau), 2,4-Diaminophenoxyethanol, 5-Amino-4-chloro-o-kresol, 3-Amino-6-methoxy-2-methylaminopyridin, 5-Amino-4-chloro-o-kresol und/oder ein physiologisch verträgliches Salz dieser Verbindungen.

8. Mittel nach einem der Ansprüche 1 bis 7, **dadurch gekennzeichnet, dass** es 2-[(2,5,6-Triaminopyrimidin-4-yl)oxy]ethanol und/oder ein physiologisch verträgliches Salze und eine oder mehrere Verbindungen, ausgewählt aus 5-Amino-2-methylphenol, 1,3-Bis-(2,4-diaminophenoxy)-propan, Resorcin, 2-Methylresorcin, 2-Amino-3-hydroxypyridin, 3-Amino-6-methoxy-2-methylaminopyridin, 2,7-Dihydroxynaphthalin sowie den physiologisch verträglichen Salzen dieser Verbindungen enthält.

9. Mittel nach einem der Ansprüche 1 bis 7, **dadurch gekennzeichnet, dass** es 6-[2-(2-Methoxyethoxy) ethoxy]pyrimidin-2-4,5-triamin und/oder ein physiologisch verträgliches Salze und eine oder mehrere Verbindungen, ausgewählt aus 5-Amino-2-methylphenol, 1,3-Bis-(2,4-diaminophenoxy)-propan, Resorcin, 2-Methylresorcin, 2-Amino-3-hydroxypyridin, 3-Amino-6-methoxy-2-methylaminopyridin, 2,7-Dihydroxynaphthalin sowie den physiologisch verträglichen Salzen dieser Verbindungen enthält.

10. Mittel zum Färben und gegebenenfalls gleichzeitigen Aufhellen keratinischer Fasern nach einem der Ansprüche 1 bis 9, dass es 0,5 bis 15 Gew.-%, vorzugsweise 1 bis 12,5 Gew.-%, besonders bevorzugt 1,5 bis 10 Gew.-% und insbesondere 2 bis 6 Gew.-% Wasserstoffperoxid, jeweils bezogen auf das Gesamtgewicht des anwendungsbereiten Mittels, enthält.

11. Verbindungen der Formel (I), ausgewählt aus-[(2,5,6-Triaminopyrimidin-4-yl)oxy]methanol, [(2,5,6-Triaminopyrimidin-4-yl)oxy]propanol, 6-(Methoxymethoxy)pyrimidin-2,4,5-triamin, 6-(2-Methoxyethoxy)pyrimidin-2,4,5-triamin, 6-(3-Methoxypropoxy)pyrimidin-2,4,5-triamin, 6-(Ethoxymethoxy)pyrimidin-2,4,5-triamin, 6-(2-Ethoxyethoxy)pyrimidin-2,4,5-triamin, 6-(3-Ethoxypropoxy)pyrimidin-2,4,5-triamin, {[(2,5,6-Triaminopyrimidin-4-yl)oxy]methoxy}methanol, {[(2,5,6-Triaminopyrimidin-4-yl)oxy]ethoxy}ethanol, {[(2,5,6-Triaminopyrimidin-4-yl)oxy]propoxy}propanol, 6-[(Methoxymethoxy)methoxy]pyrimidin-2,4,5-triamin, 6-[2-(2-Methoxyethoxy)ethoxy]pyrimidin-2,4,5-triamin, 6-[3-(3-Methoxypropoxy)propoxy]pyrimidin-2,4,5-triamin, 6-[(Ethoxymethoxy)methoxy]pyrimidin-2,4,5-triamin, 6-[2-(2-Ethoxyethoxy)ethoxy]pyrimidin-2,4,5-triamin, 6-[3-(3-Ethoxypropoxy)propoxy]pyrimidin-2,4,5-triamin, sowie deren physiologisch verträglichen Salzen.

## Claims

1. An agent for dyeing keratin fibers, in particular human hair, **characterized in that** it contains, in a cosmetic carrier, at least one compound of formula (I) and/or one physiologically acceptable salt of a compound of formula (I), 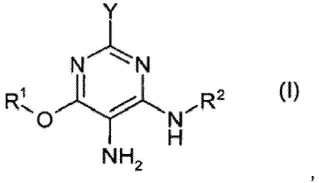 in which
R¹ can represent a C₁-C₆ hydroxyalkyl group, a C₁-C₆ alkoxy group, a C₁-C₆ alkoxy-C₁-C₆ hydroxyalkyl group, a C₁-C₆ alkoxy-C₂-C₆ alkyl group, a C₁-C₆ polyalkoxy group,
R² represents a hydrogen atom and
Y represents an NH₂ group.

2. The agent according to one of claims 1 or 2, **characterized in that** it contains at least one compound of formula (I), which is selected from the group formed by [(2,5,6-triaminopyrimidine-4-yl)oxy]methanol, [(2,5,6-triaminopyrimidine-4-yl)oxy]ethanol, [(2,5,6-triaminopyrimidine-4-yl)oxy]propanol, 6-(methoxymethoxy)pyrimidine-2,4,5-triamine, 6-(2-methoxyethoxy)pyrimidine-2,4,5-triamine, 6-(3-methoxypropoxy)pyrimidine-2,4,5-triamine, 6-(ethoxymethoxy)pyrimidine-2,4,5-triamine, 6-(2-ethoxyethoxy)pyrimidine-2,4,5-triamine, 6-(3-ethoxypropoxy)pyrimidine-2,4,5-triamine, {[(2,5,6-triaminopyrimidine-4-yl)oxy]methoxy}methanol, {[(2,5,6-triaminopyrimidine-4-yl)oxy]ethoxy}ethanol, {[(2,5,6-triaminopyrimidine-4-yl)oxy]propoxy}propanol, 6-[(methoxymethoxy)methoxy]pyrimidine-2,4,5-triamine, 6-[2-(2-methoxyethoxy)ethoxy]pyrimidine-2,4,5-triamine, 6-[3-(3-methoxypropoxy)propoxy]pyrimidine-2,4,5-triamine, 6-[(ethoxymethoxy)methoxy]pyrimidine-2,4,5-triamine, 6-[2-(2-ethoxyethoxy)ethoxy]pyrimidine-2,4,5-triamine, 6-[3-(3-ethoxypropoxy)propoxy]pyrimidine-2,4,5-triamine and the physiologically acceptable salts thereof.

3. The agent according to claim 2, **characterized in that** it contains 2-[(2,5,6-triaminopyrimidine-4-yl)oxy]ethanol and/or a physiologically acceptable salt thereof.

4. The agent according to claim 2, **characterized in that** it contains 6-[2-(2-methoxyethoxy)ethoxy]pyrimidine-2-4,5-triamine and/or a physiologically acceptable salt thereof.

5. The agent according to one of claims 1 to 4, **characterized in that** it contains the compound(s) of formula (I) and/or the physiologically acceptable salts thereof in a percentage by weight of from 0.001 to 5.0 wt.%, preferably from 0.025 to 2.5 wt.%, particularly preferably from 0.05 to 2.0 wt.%, and in particular preferably from 0.1 to 1.5 wt.%, in each case based on the total weight of the ready-to-use agent.

6. The agent according to one of claims 1 to 5, **characterized in that** it contains from 0.001 to 5.0 wt.%, preferably from 0.025 to 2.5 wt.%, particularly preferably from 0.05 to 2.0 wt.%, and in particular preferably from 0.1 to 1.5 wt.%, of at least one coupler component, all specified quantities being based on the total weight of the ready-to-use agent.

7. The agent according to claim 6, **characterized in that** it contains at least one of the following compounds as a coupler component: 1,3-bis-(2,4-diaminophenoxy)-propane, m-aminophenol, resorcinol, 5-amino-2-methylphenol, 2-methylresorcinol, 2-chloro-6-methyl-3-aminophenol, 2,7-dihydroxynaphthalene, 4-chlororesorcinol, 2,6-dihydroxy-3,4-dimethylpyridine, 1-methoxy-2-amino-4-β-hydroxy-ethylamino-benzene (Lehmann's Blue), 2,4-diaminophenoxyethanol, 5-amino-4-chloro-o-cresol, 3-amino-6-methoxy-2-methylaminopyridine, 5-amino-4-chloro-o-cresol and/or a physiologically acceptable salt of these compounds.

8. The agent according to one of claims 1 to 7, **characterized in that** it contains 2-[(2,5,6-triaminopyrimidine-4-yl)oxy]ethanol and/or a physiologically acceptable salt and one or more compounds selected from 5-amino-2-methylphenol, 1,3-bis-(2,4-diaminophenoxy)-propane, resorcinol, 2-methylresorcinol, 2-amino-3-hydroxypyridine, 3-amino-6-methoxy-2-methylaminopyridine, 2,7-dihydroxynaphthalene and the physiologically acceptable salts of these compounds.

9. The agent according to one of claims 1 to 7, **characterized in that** it contains 6-[2-(2-methoxyethoxy)ethoxy]pyrimidine-2-4,5-triamine and/or a physiologically acceptable salt and one or more compounds selected from 5-amino-2-methylphenol, 1,3-bis-(2,4-diaminophenoxy)-propane, resorcinol, 2-methylresorcinol, 2-amino-3-hydroxypyridine, 3-amino-6-methoxy-2-methylaminopyridine, 2,7-dihydroxynaphthalene and the physiologically acceptable salts of these compounds.

10. The agent for dyeing and optionally simultaneously lightening keratin fibers according to one of claims 1 to 9, in that it contains from 0.5 to 15 wt.%, preferably from 1 to 12.5 wt.%, particularly preferably from 1.5 to 10 wt.%, and in particular from 2 to 6 wt.%, of hydrogen peroxide, in each case based on the total weight of the ready-to-use agent.

11. Compounds of formula (I) selected from [(2,5,6-triaminopyrimidine-4-yl)oxy]methanol, [(2,5,6-triaminopyrimidine-4-yl)oxy]propanol, 6-(methoxymethoxy)pyrimidine-2,4,5-triamine, 6-(2-methoxyethoxy)pyrimidine-2,4,5-triamine, 6-(3-methoxypropoxy)pyrimidine-2,4,5-triamine, 6-(ethoxymethoxy)pyrimidine-2,4,5-triamine, 6-(2-ethoxyethoxy)pyrimidine-2,4,5-triamine, 6-(3-ethoxypropoxy)pyrimidine-2,4,5-triamine, {[(2,5,6-triaminopyrimidine-4-yl)oxy]methoxy}methanol, {[(2,5,6-triaminopyrimidine-4-yl)oxy]ethoxy}ethanol, {[(2,5,6-triaminopyrimidine-4-yl)oxy]propoxy}propanol, 6-[(methoxymethoxy)methoxy]pyrimidine-2,4,5-triamine, 6-[2-(2-methoxyethoxy)ethoxy]pyrimidine-2,4,5-triamine, 6-[3-(3-methoxypropoxy)propoxy]pyrimidine-2,4,5-triamine, 6-[(ethoxymethoxy)methoxy]pyrimidine-2,4,5-triamine, 6-[2-(2-ethoxyethoxy)ethoxy]pyrimidine-2,4,5-triamine, 6-[3-(3-ethoxypropoxy)propoxy]pyrimidine-2,4,5-triamine and the physiologically acceptable salts thereof.

## Revendications

1. Agent de coloration de fibres de kératine, en particulier de cheveux humains, **caractérisé en ce qu'**il contient, dans un support cosmétique, au moins un composé de formule (I) et/ou un sel physiologiquement compatible d'un composé de formule (I), 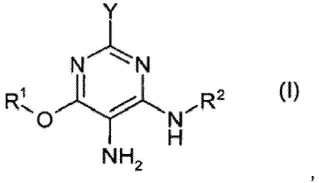 formule dans laquelle
R¹ peut représenter un groupe C₁-₆-hydroxyalkyle, un groupe C₁-₆-alcoxy, un groupe C₁-₆-alcoxy-C₁-₆-hydroxyalkyle, un groupe C₁-₆-alcoxy-C₂-₆-alkyle, un groupe C₁-₆-polyalcoxy,
R² représente un atome d'hydrogène, et
Y représente un groupe NH₂.

2. Agent selon l'une des revendications 1 ou 2, **caractérisé en ce qu'**il contient au moins un composé de formule (I) choisi dans le groupe constitué du [(2,5,6-triaminopyrimidin-4-yl)oxy]méthanol, du [(2,5,6-triaminopyrimidin-4-yl)oxy]éthanol, du [(2,5,6-triaminopyrimidin-4-yl)oxy]propanol, de la 6-(méthoxyméthoxy)pyrimidin-2,4,5-triamine, de la 6-(2-méthoxyéthoxy)pyrimidin-2,4,5-triamine, de la 6-(3-méthoxypropoxy)pyrimidin-2,4,5-triamine, de la 6-(éthoxyméthoxy)pyrimidin-2,4,5-triamine, de la 6-(2-éthoxyéthoxy)pyrimidin-2,4,5-triamine, de la 6-(3-éthoxypropoxy)pyrimidin-2,4,5-triamine, du {[(2,5,6-triaminopyrimidin-4-yl)oxy]méthoxy]méthanol, du {[(2,5,6-triaminopyrimidin-4-yl)oxy]éthoxy}éthanol, du {[(2,5,6-triaminopyrimidin-4-yl)oxy]propoxylpropanol, de la 6-[(méthoxyméthoxy)méthoxy]pyrimidin-2,4,5-triamine, de la 6-[2-(2-méthoxyéthoxy)éthoxy]pyrimidin-2,4,5-triamine, de la 6-[3-(3-méthoxypropoxy)propoxy]pyrimidin-2,4,5-triamine, de la 6-[(éthoxyméthoxy)méthoxy]pyrimidin-2,4,5-triamine, de la 6-[2-(2-éthoxyéthoxy)éthoxy]pyrimidin-2,4,5-triamine, de la 6-[3-(3-éthoxypropoxy)propoxy]pyrimidin-2,4,5-triamine ainsi que de leur sels physiologiquement compatibles.

3. Agent selon la revendication 2, **caractérisé en ce qu'**il contient du 2-[(2,5,6-triaminopyrimidin-4-yl)oxy]éthanol et/ou un de ses sels physiologiquement compatibles.

4. Agent selon la revendication 2, **caractérisé en ce qu'**il contient de la 6-[2-(2-méthoxyéthoxy)-éthoxy]pyrimidin-2-4,5-triamine et/ou un de ses sels physiologiquement compatibles.

5. Agent selon l'une des revendications 1 à 4, **caractérisé en ce qu'**il contient le ou les composés de formule (I) et/ou leurs sels physiologiquement compatibles dans une proportion en poids de 0,001 à 5,0 % en poids, de préférence de 0,025 à 2,5 % en poids, de manière particulièrement préférée de 0,05 à 2,0 % en poids et de manière tout particulièrement préférée de 0,1 à 1,5 % en poids, rapportée respectivement au poids total de l'agent prêt à l'emploi.

6. Agent selon l'une des revendications 1 à 5, **caractérisé en ce qu'**il contient 0,001 à 5,0 % en poids, de préférence de 0,025 à 2,5 % en poids, de manière particulièrement préférée de 0,05 à 2,0 % en poids et de manière tout particulièrement préférée de 0,1 à 1,5 % en poids d'au moins un composant copulateur , les données quantitatives se rapportant au poids total de l'agent prêt à l'emploi.

7. Agent selon la revendication 6, **caractérisé en ce qu'**il contient comme composant copulateur au moins un des composés suivants : le 1,3-bis-(2,4-diaminophénoxy)-propane, le m-aminophénol, le résorcinol, le 5-amino-2-méthylphénol, le 2-méthylrésorcinol, le 2-chloro-6-méthyl-3-aminophénol, le 2,7-dihydroxynaphthalène, le 4-chlororésorcinol, 2, la 6-dihydroxy-3,4-diméthylpyridine, le 1-méthoxy-2-amino-4,8-hydroxy-éthylamino-benzène (bleu de Lehmann), le 2,4-diaminophénoxyéthanol, le 5-amino-4-chloro-o-crésol, la 3-amino-6-méthoxy-2-méthylaminopyridine, le 5-amino-4-chloro-o-crésol et/ou un sel physiologiquement compatible de ces composés.

8. Agent selon l'une des revendications 1 à 7, **caractérisé en ce qu'**il contient du 2-[(2,5,6-triaminopyrimidin-4-yl)oxy]éthanol et/ou un sel physiologiquement compatible et au moins un composé choisi parmi le 5-amino-2-méthylphénol, le 1,3-bis-(2,4-diaminophénoxy)-propane, le résorcinol, le 2-méthylrésorcinol, la 2-amino-3-hydroxypyridine, la 3-amino-6-méthoxy-2-méthylaminopyridine, le 2,7-dihydroxynaphtalène ainsi que les sels physiologiquement compatibles de ces composés.

9. Agent selon l'une des revendications 1 à 7, **caractérisé en ce qu'**il contient de la 6-[2-(2-méthoxyéthoxy)-éthoxy]pyrimidin-2-4,5-triamine et/ou un sel physiologiquement compatible et au moins un composé choisi parmi le 5-amino-2-méthylphénol, 1,3-bis-(2,4-diaminophénoxy)-propane, le résorcinol, le 2-méthylrésorcinol, la 2-amino-3-hydroxypyridine, la 3-amino-6-méthoxy-2-méthylaminopyridine, le 2,7-dihydroxynaphtalène ainsi que les sels physiologiquement compatibles de ces composés.

10. Agent de coloration et éventuellement en même temps d'éclaircissement de fibres de kératine selon l'une des revendications 1 à 9, **caractérisé en ce qu'**il contient 0,5 à 15 % en poids, de préférence 1 à 12,5 % en poids, de manière particulièrement préférée 1,5 à 10 % en poids et en particulier 2 à 6 % en poids de peroxyde d'hydrogène, à chaque fois par rapport au poids total de l'agent prêt à l'emploi.

11. Composés selon la formule (I), choisis parmi le [(2,5,6-triaminopyrimidin-4-yl)oxy]méthanol, le [(2,5,6-triaminopyrimidin-4-yl)oxy]propanol, la 6-(méthoxyméthoxy)pyrimidin-2,4,5-triamine, la 6-(2-méthoxyéthoxy)pyrimidin-2,4,5-triamine, la 6-(3-méthoxypropoxy)pyrimidin-2,4,5-triamine, la 6-(éthoxyméthoxy)pyrimidin-2,4,5-triamine, la 6-(2-éthoxyéthoxy)pyrimidin-2,4,5-triamine, la 6-(3-éthoxypropoxy)pyrimidin-2,4,5-triamine, le {[(2,5,6-triaminopyrimidin-4-yl)oxy]méthoxylméthanol, le {[(2,5,6-triaminopyrimidin-4-yl)oxy]éthoxyléthanol, le {[(2,5,6-triaminopyrimidin-4-yl)oxy]propoxylpropanol, la 6-[(méthoxyméthoxy)méthoxy]pyrimidin-2,4,5-triamine, la 6-[2-(2-méthoxyéthoxy)éthoxy]pyrimidin-2,4,5-triamine, 6-[3-(3-méthoxypropoxy)propoxy]pyrimidin-2,4,5-triamine, la 6-[(éthoxyméthoxy)méthoxy]pyrimidin-2,4,5-triamine, la 6-[2-(2-éthoxyéthoxy)éthoxy]pyrimidin-2,4,5-triamine, la 6-[3-(3-éthoxypropoxy)propoxy]pyrimidin-2,4,5-triamine, ainsi que leurs sels physiologiquement compatibles.
